# FASCICULE DE BREVET EUROPEEN

(11) **EP 0 800 575 B1**
(45) Date de publication et mention de la délivrance du brevet: **14.05.2003**
(21) Numéro de dépôt: 95942764.2
(22) Date de dépôt: 18.12.1995
(51) Int. Cl.: C12N 5/10, C12N 5/08, A61K 48/00

(54) **PROCEDE DE TRANSFERT DE GENE DANS DES CELLULES ACTIVEES A PARTIR D'UN ETAT DE REPOS**
VERFAHREN DES TRANSFERS VON GENEN IN AKTIVIERTEN ZELLEN AUSGEHEND VON EINEM ZUSTAND DES TEILUNGSSTILLSTANDES
METHOD FOR GENE TRANSFER INTO CELLS ACTIVATED OUT OF A QUIESCENT STATE

(30) Priorité: 22.12.1994 FR 9415497
(43) Date de publication de la demande: 15.10.1997
(73) Titulaire: CENTRE NATIONAL DE LA RECHERCHE SCIENTIFIQUE (CNRS), 75016 Paris (FR)
(72) Inventeur: KLEIN, Antoinette, F-92160 Antony (FR); HATZFELD, Jacques, F-92160 Antony (FR)
(74) Mandataire: Grosset-Fournier, Chantal Catherine
(86) Numéro de dépôt international: FR9501691
(87) Numéro de publication internationale: WO96019567

(56) Documents cités:
- WO-A-92/11355
- WO-A-93/25216
- BLOOD (10 SUPPL. 1), vol. 84, 15 Novembre 1994 - 1994, SAUNDERS, DULUTH, NEW YORK , US, page 126A XP002001961 A. HATZFELD ET AL.: "Release from quiescence of the human hematopoietic stem cell compartment: Application in early progenitor amplification and high efficiency gene transfer"
- J. CELL BIOCHEM. SUPPL. (17 PART E), vol. 0, 1993, WILEY-LISS, NEW YORK, US, page 249 XP002001962 A. HATZFELD ET AL.: "Large scale gene transfer in early human hematopoietic progenitors"
- BRITISH JOURNAL OF HAEMATOLOGY, vol. 87, no. 1, 1994, BLACKWELL SCIENTIFIC PUBLICATIONS; OXFORD,UK, page 97 XP002001963 J. HATZFELD ET AL.: "Release from quiescence of the human hematopoietic stem cell compartment"
- PROC. NATL.ACAD SCI., vol. 90, no. 18, 15 Septembre 1993, NATL. ACAD SCI.,WASHINGTON,DC,US;, pages 8707-8711, XP002001964 A.A. CARDOSO ET AL.: "Release from quiescence of CD34+CD38- human umbilical cord blood cells reveals their potentiality to engraft adults" cité dans la demande
- LEUKEMIA, vol. 8, no. 3, Mars 1994, MACMILLAN PRESS LTD.,UK, pages 441-445, XP002001965 M.-L. LI ET AL.: "Additive effects of steel factor and antisense TGF-beta1 oligodeoxynucleotide on CD34+ hematopoietic progenitor cells"
- BLOOD CELLS, vol. 20, 1994, SPRINGER VERLAG, NEW YORK,US, pages 430-435, XP002001966 J. HATZFELD ET AL.: "Purification and release from quiescence of umbilical cord blood early progenitors reveal their potential to engraft adults"
- J. EXP. MED., vol. 178, no. 2, 1 Août 1993, ROCKEFELLER UNIV. PRESS, US, pages 529-536, XP002001967 T. MORITZ ET AL.: "Human cord blood cells as targets for gene transfer: Portential use in genetics therapies of severe combined immunodeficiency disease" cité dans la demande
- J. EXP. MED., vol. 178, no. 6, 1 Décembre 1993, ROCKEFELLER UNIV. PRESS, US, pages 2089-2096, XP002001968 L. LU ET AL.: "High efficiency retroviral mediated gene transduction into single isolated immature and replatable CD34+++ hematopoietic stem/progenitor cells from human umbilical cord blood" cité dans la demande
- COLLOQUE INSERM, vol. 229, 1993, JOHN LIBBEY EUROTEXT LTD.,PARIS, FR, pages 283-289, XP002001969 J. HATZFELD ET AL.: "Antisense oligonucleotides for inhibitors and tumor suppressor genes reveal the hematopoietic potential of quiescent progenitors"
- J. EXP. MED., vol. 174, Octobre 1991, ROCKEFELLER UNIV. PRESS, US, pages 925-929, XP002001970 J. HATZFELD ET AL.: "Release of early human hematopoietic progenitors from quience by antisense transforming growth factor beta1 or Rb oilgonucleotides" cité dans la demande
- THE LANCET, vol. 340, no. 8810, 4 Juillet 1992, THE LANCET LTD., LONDON,UK, pages 73-76, XP002001971 J.M. HOWS ET AL.: "Growth of human umbilical-cord blood in longterm haemopoietic cultures" cité dans la demande
- PROC. NATL.ACAD SCI., vol. 88, no. 19, 1 Octobre 1991, NATL. ACAD SCI.,WASHINGTON,DC,US;, pages 8377-8381, XP002001972 N. FERRY ET AL.: "Retroviral-mediated gene transfer into hepatocytes in vivo"
- NUCLEIC ACIDS RESEARCH, vol. 18 , no. 16, 25 Août 1990, IRL PRESS LIMITED,OXFORD,ENGLAND, pages 4759-4762, XP002001973 R.K. STRAIR ET AL.: "Retroviral mediated gene transfer into bone marrow progenitor cells: use of beta-galactosidase as selectable marker" cité dans la demande
- PROC. NATL.ACAD SCI., vol. 85, no. 17, Septembre 1988, NATL. ACAD SCI.,WASHINGTON,DC,US;, pages 6460-6464, XP002001974 O. DANOS ET AL.: "Safe and efficient generation of recombinant retroviruses with amphotrophic and ecotrophic host ranges" cité dans la demande
- BLOOD (1995), 86(5), 1729-35 CODEN: BLOOAW;ISSN: 0006-4971, 1995, XP000569752 SANSILVESTRI, PATRICIA ET AL: "Early CD34high cells can be separated into KIThigh cells in which transforming growth factor-.beta. (TGF-.beta.) down modulates c-kit and KITlow cells in which anti-TGF-.beta. upmodulates c-kit"
- HUM. GENE THER. (1996), 7(2), 207-13 CODEN: HGTHE3;ISSN: 1043-0342, 1996, XP000569749 HATZFELD, A. ET AL: "Increased stable retroviral gene transfer in early hematopoietic progenitors released from quiescence"

## Description

L'invention a pour objet un procédé de transfert de gène dans des cellules activées à partir d'un état de repos, notamment des cellules souches hématopoïétiques, et les cellules ainsi obtenues.

Le progrès dans l'identification et le clonage de gènes responsables de maladies génétiques humaines, a conduit à un effort important pour l'amélioration de la technologie de transfert de gène (Anderson, W.F. (1992) Science, **256:** 808-813; Miller, A.D. (1992) Nature, **357:** 455-460; Morgan, R.A., & Anderson, W.F. (1993) Annu. Rev. Biochem., **62:** 191-217; Karlsson, S. (1991) Blood, **78:** 2481-2492). Par exemple, différents vecteurs viraux ont été utilisés pour les cellules souches hématopoïétiques, chacune présentant leur propre avantage (Anderson, W.F. (1992), susmentionné). L'insertion de vecteur rétroviral dans le génome hôte assure sa réplication dans la cellule hôte. Cette insertion nécessite un état prolifératif actif (Anderson, W.F. (1992), susmentionné; Varmus, **H**.E., Padgett, T., Heasley, S., Simon, G., & Bishop, J.M. (1977), Cell.. **11:** 307-319; Nolta, J.A., & Kohn, D.B. (1990) Hum. Gene Ther., **1**: 257-268; Miller, D.G., Adam, M.A., & Miller, A.D. (1990) Mol. Cell. Biol., **10:** 4239-4242) qui n'existe pas dans le compartiment de la cellule souche hématopoïétique qui est à l'état de repos (Lajtha, L.G., & Schofield, R. (1974) Différenciation, **2:** 313-320), ou dans d'autres types de cellules souches et des cellules somatiques telles que les cellules hépatiques. En utilisant des oligonucléotides antisens contre le(s) gène(s) inhibiteur(s), en particulier TGE-β-1, on a montré que les progéniteurs de la moelle osseuse humaine précoces peuvent être relâchés de leur état de repos par blocage d'une TGF-β autocrine (Hatzfeld, J., Li, M.-L., Brown, E.L., Sookdeo, H., Levesque, J.-P., O'Toole, T., Gurney, C., Clark, S.C., & Hatzfeld, A. (1991), J. Exp. Med., **174**: 925-929).

Par ailleurs, s'agissant de greffes de tissu hématopoïétique, dans le passé, la capacité à greffer des échantillons de moelle osseuse a été analysée par l'estimation du contenu en cellules CFU-GM (colony-forming unit - granulocyte/macrophage: progéniteur granulo-monocytaire). Cependant, ces cellules qui vraisemblablement jouent un rôle important dans la génération cellulaire pendant une courte période suivant la transplantation d'un greffon, peuvent ne pas refléter le contenu en cellules plus primitives, et notamment en cellules souches hématopoïétiques qui sont importantes dans l'hématopoïèse à long terme. Les cellules souches impliquées dans des greffes à long terme représentent une petite sous population de cellules pouvant avoir éventuellement le phénotype CD34+CD38- (compartiment de la cellule souche hématopoïétique: cellules riches en antigène membranaire CD34 et pauvres en antigène membranaire de maturation CD38).

Récemment, du sang de cordon ombilical humain s'est révélé être suffisant pour reconstituer l'hématopoïèse, après la transplantation chez des enfants (Gluckman, E., Broxmeyer, H.E., Auerbach, A.D., Friedman, H.S., Douglas, G.W., Devergie, A., Esperou, H., Thierry, D., Socie, G., Lehn, P., Cooper, S., English, D., Kurtzberg, J., Bard, J., & Boyse, E.A. (1989) N. Eng. J. Med., 3: 1174-1178; Broxmeyer, H.E., Douglas, G.W., Hangoe, G., Cooper, S., Bard, J., English, D., Arny, M., Thomas, L., & Boyse, E.A. (1989) Proc. Natl. Acad. Sci. USA, **86:** 3828-3832; Broxmeyer, H.E., Hangoe, G., & Cooper, S. (1992) Bone Marrow Transplant., **9:** 7-10). *In vitro*, les données de différents laboratoires (Broxmeyer, H.E., Hangoe, G., Cooper, S., Ribeiro, R.C., Graves, V., Yoder, M., Wagner, J., Vadhan-Raj, S., Benninger, L., Rubinstein, P., & Broun, E.R. (1992) Proc. Natl. Acad. Sci. USA, **89:** 4109-4113; Lu, L., Xiao, M., Shen, R.-N., Grisby, S., & Broxmeyer, H.E. (1993) Blood, 81: 41-48; Hows, J.M., Bradley, B.A., Marsh, J.C.W., Luft, T., Coutinho, L., Testa, N.G., & Dexter, T.M. (1992) Lancet, **340:** 73-76; Cardoso, A.A., Li, M.L., Batard, P., Hatzfeld, A., Brown, E.L., Levesque, J.-P., Sookdeo, H., Panterne, B., Sansilvestri, P., Clark, S.C., & Hatzfeld, J. (1993) Proc. Natl. Acad. Sci. USA, 90: 8707-8711) suggèrent que le sang de cordon ombilical a une capacité supérieure pour générer les progéniteurs en culture, à celle de la moelle osseuse, et par conséquent, que le sang de cordon ombilical pourrait être suffisant pour effectuer des greffes de tissus hématopoïétiques sur des receveurs adultes.

Cependant, à ce jour, un transfert de gène rapide dans l'ensemble (totalité) du compartiment de la cellule souche hématopoïétique ou de toute autre cellule souche ou somatique au repos, (cet état de repos étant également désigné par "quiescence" ou comme correspondant à la phase Go), n'était pas obtenu de façon satisfaisante.

Le transfert de gène dans des cellules activées à partir d'un état de repos, notamment des cellules souches hématopoïétiques activées à partir d'un état de repos, est l'un des aspects de l'invention.

L'invention a également pour objet des cellules à l'état de repos, notamment des cellules souches hématopoïétiques à l'état de repos, contenant un gène hétérologue.

L'invention a également pour objet du tissu hématopoïétique contenant un gène hétérologue susceptible de constituer des greffons à long terme.

L'invention concerne des cellules, notamment des cellules souches hématopoïétiques, contenant dans leur génome tout ou partie d'un gène hétérologue.

L'invention concerne l'utilisation de moyens de blocage d'au moins un inhibiteur du cycle cellulaire pendant une durée n'excédant pas 72 h, et n'excédant pas de préférence 36 h, et notamment inférieure à environ 20 h, et de préférence d'environ 1 à 15 h, et de préférence d'environ 1 à 10 h, dans une culture de cellules souches, afin de libérer les cellules souches de leur état de repos.

Selon un mode de réalisation avantageux, l'invention a pour objet l'utilisation de moyens de blocage d'au moins un inhibiteur du cycle cellulaire dans un test de comparaison, d'une part, entre une culture de cellules souches dans un milieu contenant les sus-dits moyens de blocage d'au moins un inhibiteur du cycle cellulaire et, d'autre part, entre une même culture de cellules souches que celle mentionnée ci-dessus dans un milieu ne contenant pas de sus-dit moyen de blocage d'au moins un inhibiteur du cycle cellulaire, afin de déterminer le degré de maturité des sus-dites cellules souches.

Selon un autre mode de réalisation, l'invention a pour objet l'utilisation de moyens de blocage d'au moins un inhibiteur du cycle cellulaire dans une culture de cellules souches , afin de libérer les cellules souches de leur état de repos, suivie d'une étape de transfert de gènes dans les sus-dites cellules souches.

Dans le cadre de l'invention, les cellules souches sont avantageusement des cellules hématopoïétiques et les moyens de blocage d'au moins un inhibiteur du cycle cellulaire sont avantageusement constitués par de l'anti TGF-β.

L'invention concerne des cellules, notamment des cellules souches hématopoïétiques, contenant tout ou partie d'un gène hétérologue dans leur génome, susceptibles d'être obtenues selon le procédé comprenant les étapes suivantes:
- la préstimulation de cellules au repos, notamment de cellules souches hématopoïétiques au repos dans un milieu contenant:
   * des moyens de blocage directs ou indirects d'au moins un inhibiteur du cycle cellulaire des dites cellules, notamment des dites cellules souches hématopoïétiques, pendant une durée suffisante pour libérer les cellules, notamment les cellules souches hématopoïétiques, de leur état de repos, et n'excédant pas de préférence 72 h, et n'excédant pas de préférence 36 h, et est notamment inférieure à environ 20 h, et est de préférence d'environ 1 h à 15 h, et de préférence d'environ 1 h à 10 h, et
   * des substances, notamment des cytokines, pour permettre l'activation d'un cycle cellulaire et/ou ultérieurement la traversée d'au moins un cycle cellulaire,
- l'étape de transfert par la mise en présence des cellules, notamment des cellules souches hématopoïétiques, obtenues à l'étape précédente avec tout ou partie d'un gène hétérologue, dans un milieu permettant le transfert de tout ou partie du gène hétérologue dans le génome des cellules, notamment des cellules souches hématopoïétiques, pour obtenir des cellules, notamment des cellules souches hématopoïétiques, comportant tout ou partie d'un gène hétérologue,
- la récupération éventuelle des cellules, notamment des cellules souches hématopoïétiques, obtenues à l'étape précédente.

L'invention concerne plus particulièrement des cellules souches hématopoïétiques contenant dans leur génome tout ou partie d'un gène hétérologue et ayant un potentiel de multiplication *in vitro* égal ou supérieur à 106 et un potentiel de différenciation susceptible de couvrir tous les lignages hématopoïétiques.

Les cellules souches hématopoïétiques sont définies comme des cellules au repos pouvant donner naissance à tous les lignages hématopoïétiques et aux cellules en dérivant. Quelle que soit l'origine des cellules souches (foie foetal, moelle foetale, sang de cordon ombilical, moelle adulte, sang périphérique) leur potentiel de prolifération est toujours très élevé: une cellule donnant naissance à plus de 1 à 20 x 10⁶ cellules *in vitro*. Ce nombre peut être plus élevé si l'on utilise de nouvelles cytokines et des bioréacteurs.

Les cellules souches hématopoïétiques peuvent provenir d'échantillon de moelle osseuse originaire d'une ponction de la crête iliaque sur des donneurs de moelle osseuse.

Les cellules souches hématopoïétiques peuvent également être récupérées à partir de sang de cordon ombilical prélevé immédiatement après la naissance, de foie ou de moelle foetale de sang périphérique ou de tout autre organe du système hématopoïétique.

Un potentiel de multiplication égal ou supérieur à 10⁶ signifie qu'une cellule souche hématopoïétique peut donner, dans des conditions appropriées, 10⁶ cellules du sang.

Le potentiel de multiplication des cellules souches hématopoïétiques de l'invention peut atteindre 1 à 20 x 10⁶ cellules en milieu Stem GEM* et en culture traditionnelle. Cette valeur peut être élevée si l'on utilise de nouvelles cytokines et des bioréacteurs.

Par lignages hématopoïétiques, on désigne l'ensemble des cellules donnant naissance à l'un des différents types cellulaires du sang.

Par cellules au repos susceptibles de rentrer dans le cadre de l'invention, on désigne des cellules qui ne se multiplient pas en situation physiologique ordinaire et sont susceptibles d'être activées en culture. Comme exemple, on peut citer: les cellules du foie, du tissu osseux, des cellules endothéliales, du tissu du système nerveux, etc..., ou des cellules souches hématopoïétiques qui restent pour la majorité d'entre elles au repos chez l'individu normal.

Selon un mode de réalisation avantageux, l'invention concerne des cellules, notamment des cellules souches hématopoïétiqués, comportant, à l'intérieur d'elles mêmes ou sur leur surface, des moyens de blocage de tout inhibiteur de leur cycle cellulaire.

Comme moyens de blocage d'inhibiteurs du cycle cellulaire à l'intérieur de la cellule, on peut citer des oligonucléotides antisens, des ribozymes, des inhibiteurs chimiques naturels ou de synthèse, des anticorps bloquants, des cytokines telles que le TNF, des interférons ou des facteurs tels que le MIP-1α, le facteur 4 plaquettaire ou le FGF. Ceux-ci peuvent pénétrer naturellement avec des milieux de cultures appropriées ou par électroporation, par microinjection ou par tout autre procédé utilisant un transport à travers la membrane tels que les liposomes.

Comme moyens de blocage d'inhibiteurs du cycle cellulaire sur la surface de la cellule, on peut citer des anticorps de récepteurs ou d'antigènes membranaires, des ligands de récepteur, naturels ou de synthèse, des combinaisons de cytokines comprenant diverses interleukines et Colony Stimulating Factors, CSFs (facteurs stimulant les colonies) ou facteurs de croissance hématopoïétiques.

Les anticorps et les ligands se fixent spécifiquement sur un antigène ou un récepteur qui leur correspond.

Dans les cellules de l'invention, notamment dans les cellules souches hématopoïétiques, les moyens de blocage peuvent être constitués par ceux choisis parmi: les anti-inhibiteurs du cycle cellulaire, par exemple des agents bloquant les gènes suppresseurs de tumeurs, des antagonistes du TGF-β tels que: des oligonucléotides antisens du TGF-β, des anticorps neutralisants le TGF-β, des récepteurs solubles du TGF-β ou des inhibiteurs naturels ou de synthèse du TGF-β.

L'abréviation TGF-β correspond à "transforming growth factor" (facteur de croissance transformant) et correspond à une famille de protéines.

Comme exemple d'antigènes suppresseurs de tumeurs, on peut citer pRb, p107 (Zhu et al., Genes and development, **7:** 1111-1125, 1993), p130 (Hannon et al., Genes Dev., **7:** 2378-2391, 1993), p300, p53, p15 (Hannon G.J. & Beach D. (1994): "p15^{INK4B} is a potential effector of TGF-β-induced cell cycle arrest", Nature, **371:** 257) , p16 (Ivison A.J. (1994): "p16 and familial melanoma". Nature Genet., 371: 180, Sansilvestri P. Thèse de Doctorat d'Etat Paris XI, 1994), p21 (Xiong Y., et al., & Beach D. (1993): "p21 is a universal inhibitor of cyclin kinases". Nature, 366: 701), p27 (Polyak et al. (1994a): Cloning of p27^{kip1}, a cyclin-dependent kinase inhibitor and a potential mediator of extracellular antimitogenic signals". Cell, **78:** 59-66; Polyak et al. (1994a): p27^{kip1}, a cyclin-cdk inhibitor, links transforming growth factor b and contact inhibition to cell cycle arrest". Genes Development, **8:** 9-22).

Par antagonistes du TGF-β, on désigne un agent qui neutralise *in vitro* ou *in vivo* l'activité biologique du TGF-β, types 1, 2 ou 3.

Par anticorps neutralisants, on désigne un anticorps ou une combinaison d'anticorps, de préférence un anticorps monoclonal ou une combinaison d'anticorps monoclonaux, qui neutralise *in vitro* ou *in vivo* l'activité biologique du TGF-β de types 1, 2 ou 3.

Par inhibiteur naturel ou de synthèse du TGF-β, on désigne une substance qui inhibe *in vitro* ou *in vivo* l'activité biologique du TGF-β.

Comme nucléotide antisens du TGF-β, on peut avantageusement utiliser:

5'-CCCGGAGGGCGGCATGGGGA-3'

Comme moyens de blocage, on peut également envisager une cytokine ou une combinaison de cytokines susceptibles de libérer les cellules souches de l'état de repos pendant une durée inférieure à environ 10 h.

Comme cytokines, on peut citer: toutes les interleukines, tous les CSF, (facteurs stimulant les colonies) tous les immunomodulateurs (par exemple les interleukines suivantes: IL1, IL2, IL3, IL4, ILS, IL6, IL7, IL8, IL9, IL10, IL11, IL12, IL13, les TNF (Tumor Necrosis Factors: Facteurs Nécrosant de Tumeur), les interférons, le LIF (facteur inhibiteur de leucémie), le MIP-1α (macrophage inflammatory protein: protéine inflammatoire produite ou agissant sur le macrophage, Dunlop D.J. et al.: Blood, 1992, 79: 2221-2225), le Steel Factor ou Facteur Steel, le ligand du récepteur flt-3 ou FL.

Comme combinaison de cytokines, on peut citer l'IL3, l'IL6, le GMCSF (facteur de croissance des progéniteurs granulo-monocytaires), le SF et le FL.

Les moyens de blocage de tout inhibiteur du cycle cellulaire doivent être utilisés en quantité appropriée pour neutraliser l'activité de tout inhibiteur du cycle cellulaire. Ils sont généralement utilisés à des concentrations allant de 0,1 à 1000 unités/ml, de préférence de 1 à 10 unités/ml.

Lorsqu'on utilise un oligonucléotide antisens du TGF-β, celui-ci peut être utilisé de 0,03 µM à 100 µM, notamment de 5 µM à 8 µM, ou de 0,01 µM à 0,5 µM en présence de certains agents par exemple lipidiques permettant une meilleure pénétration des oligonucléotides dans la cellule que les pénétrations précédemment décrites.

Selon un mode de réalisation avantageux, l'invention concerne des cellules à l'état de repos, notamment des cellules souches hématopoïétiques contenant, intégré dans leur génome, tout ou partie d'un gène hétérologue.

En fait, après le transfert de tout ou partie d'un gène hétérologue dans leur patrimoine génétique, les cellules peuvent être replacées à l'état de repos.

Le procédé selon lequel, après transfert de tout ou partie d'un gène hétérologue, les cellules sont replacées à l'état de repos, est détaillé dans la suite de la présente description.

L'invention concerne également une composition de cellules hématopoïétiques ou un amas de cellules hématopoïétiques ou un tissu de cellules hématopoïétiques, susceptibles de maintenir une hématopoïèse continue pendant une durée supérieure ou égale à environ 6 mois, et constitués par des cellules souches hématopoïétiques et le cas échéant des progéniteurs en cours de différenciation, les susdites cellules souches et les susdits progéniteurs contenant tout ou partie d'un gène hétérologue.

L'expression "maintenir une hématopoïèse continue" correspond à une production physiologique de cellules matures du sang.

L'invention concerne également un procédé de transfert de tout ou partie d'un gène hétérologue dans le génome de cellules activées à partir d'un état de repos, notamment de cellules souches hématopoïétiques activées à partir d'un état de repos, caractérisé en ce qu'il comprend les étapes suivantes:
- la préstimulation de cellules au repos, notamment de cellules souches hématopoïétiques au repos dans un milieu de préstimulation contenant:
   * des moyens de blocage directs ou indirects d'au moins un inhibiteur du cycle cellulaire des dites cellules au repos, notamment des dites cellules souches hématopoïétiques pendant une durée suffisante pour libérer les cellules, notamment les cellules souches hématopoïétiques de leur état de repos, et n'excédant pas de préférence environ 72 h, n'excédant pas de préférence 36 h, et est notamment inférieure à environ 20 h et est de préférence d'environ 1 h à 15 h, et de préférence d'environ 1 h à 10 h, et :
      * des substances, notamment des cytokines, pour permettre l'activation d'un cycle cellulaire et/ou ultérieurement la traversée d'au moins un cycle cellulaire des dites cellules, notamment des dites cellules souches,
- l'étape de transfert par la mise en présence des cellules, notamment des cellules souches hématopoïétiques préstimulées obtenues à l'étape précédente avec tout ou partie d'un gène hétérologue, dans un milieu permettant le transfert de tout ou partie du gène hétérologue dans le génome des cellules, notamment des cellules souches hématopoïétiques, pour obtenir des cellules, notamment des cellules souches hématopoïétiques comportant tout ou partie d'un gène hétérologue,
- la récupération éventuelle des cellules, notamment des cellules souches hématopoïétiques, obtenues à l'étape précédente.

Les cellules souches hématopoïétiques au repos utilisées dans le cadre du transfert de gène peuvent être obtenues à partir d'organes hématopoïétiques foetaux, de sang de cordon ombilical, de moelle osseuse, de sang périphérique ou de tout autre organe hématopoïétique, selon le procédé décrit dans les exemples (Hatzfeld J., Batard P., Cardoso A.A., Li M.-L., et Hatzfeld A.: "Purification and *in vitro* assay of early human hematopoietic progenitors", 205-221. Dans Culture of Hematopoietic Cells. Freshney R.I., Pragnell I.B., Freshney M.G., 1994).

En ce qui concerne la durée de la préstimulation, elle n'excède pas environ 72 h, de préférence 36 h, et est de préférence d'environ 1 h à 15 h, et de préférence d'environ 1 h à 10 h.

L'induction de la préstimulation peut se faire pendant un temps extrêmement court, de l'ordre de quelques secondes, mais le déclenchement de l'ensemble des mécanismes cellulaires permettant à la cellule souche de sortir de la phase Go prennent au moins 1 h.

Dans le cas de l'utilisation, comme moyen de blocage, d'anti-TGF-β, la préstimulation n'excède pas environ 72 h, car au-delà de 72 h, et notamment au-delà de 19 h, on n'a pas d'effet supplémentaire à la préstimulation obtenue avec des combinaisons de cytokines ou l' anti-TGF-β et des combinaisons de cytokines.

L'expression "activation d'un cycle cellulaire" correspond à la mise en place des fonctions permettant la traversée du cycle cellulaire.

L'expression "la traversée d'au moins un cycle cellulaire" correspond à une division du génome (phase S), et à une mitose donnant deux cellules identiques.

A l'issue du transfert de gène, les cellules peuvent, soit se multiplier et se différencier, soit peuvent être replacées à l'état de repos, comme il est explicité dans la suite de la description.

Lorsque l'on replace les cellules souches transfectées à l'état de repos, le compartiment de la cellule souche a "vieilli" d'une division ce qui est peu par rapport à son énorme potentiel hématopoïétique.

Dans le procédé de l'invention, selon un mode de réalisation avantageux le milieu de préstimulation comprend: IMDM (Iscove Dubelcco Modified Medium), (StemGEM* correspondant à une gamme de milieux commercialisés par le C.N.R.S.), des cytokines, des antagonistes du TGF-β tels que des oligonucléotides antisens du TGF-β, des anticorps neutralisants le TGF-β, des récepteurs solubles du TGF-β ou des inhibiteurs naturels ou de synthèse du TGF-β, des combinaisons de cytokines susceptibles de bloquer dans un espace de temps court (inférieur à 72 h, de préférence inférieur à 10 h), les inhibiteurs du cycle cellulaire des cellules souches quiescentes, contrôlés ou non par le TGF-β.

Selon un mode de réalisation du procédé de l'invention, les cytokines sont choisies parmi une combinaison d'au moins quatre cytokines choisies parmi les cytokines suivantes: IL1 (interleukine 1), IL2 (interleukine 2), IL3 (interleukine 3), IL4 (interleukine 4), ILS (interleukine 5), IL6 (interleukine 6), IL7 (interleukine 7), IL11 (interleukine 11), GM-CSF (granulocyte-macrophage colony-stimulating factor: facteur de croissance des progéniteurs granulo-monocytaires), G-CSF (granulocyte colony-stimulating factor: facteur de croissance des progéniteurs granulocytaires), facteur Steel (SF), ligand du récepteur FLT3 (FL), érythropoïétine, TNF, LIF (leukemia inhibitor factor: facteur inhibiteur de la leucémie), thrombopoïétine, insulines et FGF.

Le transfert de tout ou partie d'un gène hétérologue peut se faire avec une coculture de cellules, notamment des cellules souches hématopoïétiques, préstimulées selon la première étape du procédé de l'invention, et de cellules produisant un vecteur, tel qu'un vecteur viral ou un rétrovirus, contenant tout ou partie du gène à transférer. (Ferry N., Duplessis O., Houssin D., Danos O., et Heard J.-M. "Retroviral-mediated gene transfer into hepatocytes *in vivo".* Proc. Natl. Acad. Sci., USA, **88:** 8377-8381, 1991).

Le transfert peut également être effectué en remettant en présence des cellules, notamment des cellules souches hématopoïétiques, préstimulées et du surnageant de cellules contenant un vecteur, tel qu'un vecteur viral ou un rétrovirus comprenant tout ou partie du gène à transférer (Lu, R.L., Xiao, M., Clapp, D.-W., Li, Z.-H., & Broxmeyer, H.E. (1993) J. Exp. Med., **178:** 2089-2096).

L'invention concerne également un procédé de transfert de tout ou partie d'un gène hétérologue dans le génome de cellules activées à partir d'un état de repos, notamment de cellules souches hématopoïétiques activées à partir d'un état de repos, caractérisé en ce qu'il comprend les étapes suivantes:
- la préstimulation de cellules au repos, notamment de cellules souches hématopoïétiques au repos dans un milieu de préstimulation contenant:
   * des moyens de blocage directs ou indirects d'au moins un inhibiteur du cycle cellulaire des dites cellules, notamment des cellules souches hématopoïétiques pendant une durée suffisante pour libérer les cellules, notamment les cellules souches hématopoïétiques de leur état de repos, et n'excédant pas de préférence environ 72 h, et n'excédant pas de préférence environ 36 h, et est notamment inférieure à environ 20 h et est de préférence d'environ 1 h à 15 h, et de préférence d'environ 1 h à 10 h, et
   * des substances, notamment des cytokines, pour permettre l'activation d'un cycle cellulaire et/ou ultérieurement la traversée d'au moins un cycle cellulaire des dites cellules, notamment des dites cellules souches hématopoïétiques,
- l'étape de transfert par la mise en présence des cellules, notamment des cellules souches hématopoïétiques, préstimulées obtenues à l'étape précédente avec tout ou partie d'un gène hétérologue, dans un milieu permettant le transfert de tout ou partie du gène hétérologue dans le génome des cellules souches, par coculture de cellules, notamment de cellules souches hématopoïétiques préstimulées comme indiqué ci-dessus et de cellules produisant un vecteur viral ou un rétrovirus contenant tout ou partie du gène à transférer, pour obtenir des cellules, notamment des cellules souches hématopoïétiques comportant tout ou partie d'un gène hétérologue,
- la récupération éventuelle des cellules, notamment des cellules souches hématopoïétiques obtenues à l'étape précédente.

La coculture peut être effectuée comme décrit dans Human Cord Blood Cells as Targets for Gene Transfer: Potential Use in Genetic Therapies of Severe Combined Immunodeficiency Disease. Moritz T., Keller D.C. et Williams D.A., J. Exp. Med., 178: 529-536 (1993).

L'invention concerne également un procédé de transfert de tout ou partie d'un gène hétérologue dans le génome de cellules activées à partir d'un état de repos, notamment de cellules souches hématopoïétiques activées à partir d'un état de repos, caractérisé en ce qu'il comprend les étapes suivantes:
- la préstimulation de cellules au repos, notamment de cellules souches hématopoïétiques au repos dans un milieu de préstimulation contenant:
   * des moyens de blocage directs ou indirects d'au moins un inhibiteur du cycle cellulaire des dites cellules, notamment des dites cellules souches hématopoïétiques, pendant une durée suffisante pour libérer les cellules, notamment les cellules souches hématopoïétiques de leur état de repos, et n'excédant pas de préférence environ 72 h, et n'excédant pas de préférence environ 36 h, et est notamment inférieure à environ 20 h et est de préférence d'environ 1 h à 15 h, et de préférence d'environ 1 h à 10 h, et
   * des substances, notamment des cytokines, pour permettre l'activation d'un cycle cellulaire et/ou ultérieurement la traversée d'au moins un cycle cellulaire des dites cellules souches,
- l'étape de transfert par la mise en présence des cellules, notamment des cellules souches hématopoïétiques, préstimulées obtenues à l'étape précédente avec tout ou partie d'un gène hétérologue, dans un milieu permettant le transfert de tout ou partie du gène hétérologue dans le génome des cellules, notamment des cellules souches hématopoïétiques, par mise en présence des cellules, notamment des cellules souches préstimulées comme indiqué ci-dessus et du surnageant de cellules contenant un vecteur, tel qu'un vecteur viral ou un rétrovirus contenant tout ou partie du gène à transférer, pour obtenir des cellules, notamment des cellules souches comportant tout ou partie d'un gène hétérologue,
- la récupération éventuelle des cellules, notamment des cellules souches obtenues à l'étape précédente.

L'invention concerne également un procédé de transfert de tout ou partie d'un gène hétérologue dans le génome de cellules activées à partir d'un état de repos, notamment de cellules souches hématopoïétiques activées à partir d'un état de repos, caractérisé en ce qu'il comprend les étapes suivantes:
- la préstimulation de cellules au repos, notamment de cellules souches hématopoïétiques au repos dans un milieu de préstimulation contenant:
   * des moyens de blocage d'au moins un inhibiteur du cycle cellulaire des dites cellules, notamment des dites cellules souches hématopoïétiques, pendant une durée suffisante pour libérer les cellules, notamment les cellules souches hématopoïétiques de leur état de repos, et n'excédant pas de préférence environ 72 h, et est notamment inférieure à environ 20 h et est de préférence d'environ 1 h à 15 h, et de préférence d'environ 1 h à 10 h, et
   * des substances, notamment des cytokines, pour permettre l'activation d'un cycle cellulaire et/ou ultérieurement la traversée d'au moins un cycle cellulaire des dites cellules, notamment des dites cellules souches hématopoïétiques,
- l'étape de transfert par la mise en présence des cellules, notamment des cellules souches hématopoïétiques, préstimulées obtenues à l'étape précédente avec tout ou partie d'un gène hétérologue, dans un milieu permettant le transfert de tout ou partie du gène hétérologue dans le génome des cellules, notamment des cellules souches hématopoïétiques, pour obtenir des cellules, notamment des cellules souches hématopoïétiques comportant tout ou partie d'un gène hétérologue, tout ou partie du gène hétérologue provenant notamment d'un vecteur, tel qu'un vecteur viral ou un rétrovirus, produit par une culture productrice à laquelle a été additionné un agent viral, tel qu'un anti-TGF-β, un anti-interféron ou tout agent permettant d'augmenter la production et/ou la stabilité du vecteur,
- la récupération éventuelle des cellules, notamment des cellules souches hématopoïétiques obtenues à l'étape précédente.

S'agissant des cellules souches, elles sont éventuellement remises à l'état de repos après l'étape de transfert.

Selon un mode de réalisation avantageux du procédé de l'invention, le milieu permettant le transfert de tout ou partie du gène hétérologue est constitué par, ou comprend: DMEM (milieu de Eagle modifié par Dubelcco), et éventuellement IMDM (milieu d'Iscove modifié par Dubelcco), des cytokines, et éventuellement un antagoniste du TGF-β tel que défini ci-dessus, des anti-interférons, ou tout autre agent viral, permettant d'augmenter la production et/ou la stabilité du vecteur viral ou du rétrovirus, contenant le gène à transfecter, lorsque le transfert de tout ou partie du gène hétérologue se fait par coculture des cellules souches et du vecteur viral, ou du rétrovirus.

Lorsque le transfert se fait en utilisant un surnageant de la culture contenant le vecteur, le titre du vecteur produit par la culture productrice peut être avantageusement augmenté en ajoutant à la culture productrice du vecteur, un anti-TGF-β ou un anti-interféron ou tout autre agent susceptible d'augmenter la production et/ou la stabilité du vecteur.

L'invention concerne également un procédé permettant d'augmenter le titre d'un vecteur, tel qu'un vecteur viral, ou un rétrovirus, lequel vecteur est produit par une culture productrice, par addition à ladite culture d'un agent viral tel qu'un ami-TGF-β, un anti-interféron, ou de tout agent stabilisateur de vecteur.

A l'issue de l'étape du transfert de tout ou partie du gène hétérologue dans les cellules souches, les cellules souches comportant dans leur génome tout ou partie d'un gène hétérologue peuvent être placées dans un milieu approprié, notamment un milieu liquide ou un milieu semi-solide, afin de se multiplier et de se différencier en des cellules hématopoïétiques différenciées, notamment en cellules différenciées du sang.

Comme exemple de cellules différenciées, on peut citer les granulocytes, les monocytes, les mégacaryocytes, les érythrocytes.

A l'issue de l'étape de transfert de tout ou partie du gène hétérologue dans les cellules hématopoïétiques, on peut envisager avantageusement une étape de remise à l'état de repos des cellules souches hématopoïétiques contenant tout ou partie du gène hétérologue, cette étape pouvant être effectuée à l'aide d'au moins un inhibiteur du cycle cellulaire, tel que le TGF-β.

Plus précisément, pour remettre les cellules souches au repos (c'est-à-dire en phase Go), après transfert de tout ou partie d'un gène hétérologue, on peut procéder de la façon suivante.

L'intérêt de la méthode de l'invention sur toutes les autres méthodes tient au fait que l'activation de la cellule au repos se fait dans un laps de temps extrêmement court (de l'ordre de 1 à 72 heures, de préférence 6 à 12 heures à partir de la fin de l'étape de préstimulation) grâce à une synchronisation de la sortie de la phase Go pour toutes les cellules au repos lors de l'étape de préstimulation du procédé de l'invention tel que décrit ci-dessus. Cela permet de remettre les cellules transfectées en phase Go, après le transfert par addition de TGF-β. Ceci garantit que pendant le transfert de gène(s) les cellules souches n'ont pas perdu leur potentiel hématopoïétique, dans la mesure où le nombre de division n'est pas supérieur à 1 ou 2.

Le gène hétérologue dont tout ou partie est introduit dans le génome de cellules souches hématopoïétiques peut être choisi parmi les gènes suivants: marqueurs: Neo (gène de résistance à la néomycine), CD2 (antigène leucocytaire), nls-LacZ, ou des gènes corrigeant une hématopathie ou tout autre pathologie telle que ADA (gène de l'adénosine déaminase), ALDP (gène corrigeant l'adrénoleucodystrophie), TK (gène suicide permettant de tuer des cellules en présence d'acyclovir sous le contrôle de promoteurs viraux), cette liste n'étant pas limitative.

Les méthodes actuelles de transfert de gène, ne permettent pas de transfecter le compartiment de la cellule souche de façon à ce que les lignages lymphoïdes soient transfectés de façon satisfaisante. Or, ceci est important si l'on veut que la thérapie cellulaire soit applicable au traitement du SIDA ou de pathologies concernant les cellules B (lymphomes etc.....). Le procédé de transfert de l'invention permet effectivement le transfert dans les lignages B et T, ce qui peut être vérifié en utilisant les deux modèles suivants permettant d'étudier le développement du compartiment de la cellule souche:
- des souris SCID humanisées (McCune J.M., Namikawa R., Weilbaecher K.N., Kaneshima H., Schultz L.D., Lieberman M., et Weissman IL.: "The SCID-hu mouse: Murine model for the analysis of human hematolymphoid differentiation and function". Science, **241**: 1632, 1988).
- des cultures d'épithélium thymique (Plum J., De Smedt M., Defresne M.-P., Leclercq G., et Vandekerckhove B.: "Human CD34+ fetal liver stem cells differentiate to T cells in a mouse thymic microenvironment". Blood 84: 1587-1593, 1994).

Ces méthodes permettent d'obtenir dans le premier cas, tous les lignages y compris B et T, dans le deuxième cas, le lignage T.

Les milieux utilisés pour permettre la division du compartiment de la cellule souche une fois activée pour sortir de la phase Go, doivent respecter l'expression des récepteurs de la cellule souche selon des recommandations publiées dans Hatzfeld A. et al., Hématol., 1993, **35:** 281-283, et dans Panterne et al., J. Cell. Physiol., **91,** 1481:1489.

Le procédé de l'invention permet notamment:
- d'augmenter le titre d'un vecteur contenant tout ou partie d'un gène hétérologue à transfecter, notamment dans des cellules souches hématopoïétiques,
- de transférer tout ou partie d'un gène dans des cellules au repos, notamment des cellules souches hématopoïétiques, dans lesquelles, jusqu'à présent un tel transfert était aléatoire;
- d'améliorer l'efficacité de transfert dans les progéniteurs hématopoïétiques précoces tels que CFU-GEMM, LTC-IC (cellule initiatrice de culture à long terme: long term culture-initiating cell), HPP-Q (High Proliferative Potential Quiescent cells = cellules au repos à haut pontentiel prolifératif) et progéniteurs lymphoïdes à fort potentiel prolifératif considérés comme "le compartiment de la cellule souche" par rapport au transfert déjà réalisé en mettant en oeuvre les procédés de l'art antérieur;
   par exemple, la méthode permettra un transfert dans plus de 95% des CFU-GEMM pouvant se développer en colonies contenant plus de 10⁵ cellules, les milieux Stem GEM* distribués par le CNRS permettant d'évaluer de telles cellules du compartiment de la cellule souche, les souris SCID (souris immunodéficientes: severe combined immunodeficiency) humanisées et des systèmes de cultures stromales ou d'épithélium thymique (référencées ci-dessus) permettant d'évaluer le transfert dans tout le compartiment de la cellule souche;
- d'améliorer la stabilité de l'expression au cours du développement des cellules souches et des progéniteurs (amplification et différenciation), des gènes marqueurs pouvant être utilisés pour suivre et évaluer cette expression (exemple: nls-Lac-Z).

L'invention concerne également un procédé permettant d'évaluer la maturité ou l'immaturité de cellules souches, avant et/ou après le transfert de gènes.

L'invention concerne plus particulièrement un procédé de détermination du degré de maturité de cellules souches, notamment hématopoïétiques, caractérisé en ce qu'il comprend les étapes suivantes :
- on effectue la culture d'un premier ensemble de cellules souches, notamment hématopoïétiques, dans un milieu approprié à leur culture, mais ne contenant pas de moyens de blocage d'au moins un inhibiteur du cycle cellulaire, pendant environ 14 à environ 28 jours, de préférence 18 jours,
- on effectue la culture d'un deuxième ensemble de cellules souches, notamment hématopoïétiques, de même nature et de même degré de maturité que celles mentionnées ci-dessus, dans un milieu approprié contenant des moyens de blocage d'au moins un inhibiteur du cycle cellulaire, ces moyens de blocage étant présents, dans le milieu de culture à une concentration efficace (à condition de ne pas être diluée plus de 4 fois par rapport à la concentration optimale), pendant une période n'excédant pas 72 h, et n'excédant pas de préférence environ 36 h, et est de préférence d'environ 1 à 15 h et de préférence encore, de 1 à 10 h, cette culture ayant lieu pendant la même durée que celle du premier ensemble de cellules souches,
- on compare, 18 jours après la mise en culture de chacun des deux ensembles de cellules souches, le nombre et la nature des colonies, la différence des sommes des colonies à haut potentiel prolifératif (HPP-CFC = cellule formant une colonie à haut potentiel prolifératif, HPP-MEG = mégacaryocyte à haut potentiel prolifératif, HPP-GEMM = granulocyte érythrocyte monocyte mégacaryocyte à haut potentiel prolifératif) respectivement entre celle du deuxième ensemble et celle du premier ensemble correspondant aux colonies HPP-Q.

La concentration optimale d'oligonucléotides anti-sens peut être d'environ 0,001 µM à environ 100 µM, de préférence inférieure à environ 10 µM.

La concentration optimale en anticorps bloquants peut être d'environ 0,01 µg/ml à environ 100 µg/ml, de préférence 1 µg/ml.

S'agissant de la culture en présence de moyens de blocage d'un inhibiteur du cycle cellulaire, à l'issue de la période n'excédant pas 72 h, et n'excédant pas de préférence environ 36 h, et qui est de préférence d'environ 1 à 15 h et de préférence encore, de 1 à 10 h :
- soit on peut laver les cellules pour éliminer substantiellement les sus-dits moyens de blocage,
- soit on peut diluer les sus-dits moyens de blocage à une concentration telle qu'ils soient sans effet prolifératif sur des cellules plus matures (c'est-à-dire progéniteurs déjà activés).

"L'évaluation de la maturité ou de l'immaturité de cellules souches" correspond à l'observation suivante.

Après 18 jours, on observe au microscope le nombre et la nature des colonies: on compte le nombre de colonies mixtes constituées de cellules de la lignée rouge et d'un ou plusieurs types de cellules de la lignée blanche. Le nombre de colonies mixtes est plus élevé lorsque la population cellulaire mise en culture est plus immature, c'est-à-dire constituée de cellules plus jeunes dont certaines sont au repos. D'autre part, plus la cellule qui a donné naissance à la colonie est immature, voir au repos, plus son potentiel de prolifération est grand, donc la colonie provenant de cette cellule une fois activée sera plus importante en taille.

Ce test sera également désigné dans la suite par test HPP-Q.

Pour ce faire, on compare une culture de cellules souches dans un milieu semi-solide ou liquide ne contenant pas d'anti-inhibiteur du cycle cellulaire avec la même culture de cellules souches dans un milieu semi-solide ou liquide contenant un anti-inhibiteur du cycle cellulaire, par exemple de l'anti-TGF-β.

Les conditions de culture dans le milieu ne contenant pas d'anti-inhibiteur de cycle cellulaire peuvent utiliser l'IL1, l'IL2, l'IL3, l'IL4, l"IL5 l'IL6, l'IL7, l'IL11, le G-CSF, le GM-CSF, le SF, le FL, le FGF, l'insuline, l'érythropoïetine, la thrombopoïetine où d'autres cytokines qui coopèrent ou synergisent avec les précédentes.

Les conditions de culture dans le milieu contenant un anti-inhibiteur du cycle cellulaire par exemple l'anti-TGF-β sont les suivantes : l'anti-TGF-β n'a été ajouté qu'en préstimulation pendant 10 heures dans un faible volume de culture liquide contenant les cellules au repos telles les cellules CD34+CD38-(1 à 10% des CD34+ les plus immatures). Le volume de cette préculture est de 1/6 environ du volume final. Cette préculture est ensuite ajoutée à de la méthylcellulose avec des cytokines non additionnées d'anti-TGF-β, pour aboutir au volume final.

Une simplification de cette procédure consiste à mettre l'anti TGF-β immédiatement dans la culture finale à des concentrations qui ne lui permettent d'être actif que pendant les premières heures de la culture, de préférence pendant les 10 premières heures.

Dans ces conditions, on observe de nouvelles colonies plus grosses que les précédentes, dérivées de cellules appelées High Proliferative Potential Quiescent Cells ou HPP-Q qui se développent sur 18 jours et non pas seulement sur 8, 10, 12 ou 14 jours comme on l'observe sans préstimulation par l'anti-TGF-β. L'anti-TGF-β n'a donc pas besoin d'être présent pendant toute la durée de la culture. S'agissant de la taille des colonies, elle peut être déterminée par comptage au microscope sur une lame de Mallasez, après prélèvement et dispersion de la colonie dans un volume connu de milieu liquide ; cette méthode permet d'évaluer le nombre de cellules constituant la colonie.

Que le test soit fait en milieu liquide, clonal ou non clonal, on compare les clones ou les populations dans les deux types de culture.

S'il s'agit des HPP-Q, elles constituent un élément du compartiment de la cellule souche et, quant à leur phénotypage, il s'agit de CD34+ Kit Low IL6-R low.

S'agissant des milieux de culture, on peut utiliser tout milieu approprié pour les cellules CFU-GEMM et permettant également le développement des cellules lymphoïdes.

La composition des milieux de culture semi-solide peut être telle qu'elle est décrite dans le chapitre 12, inclus dans le livre intitulé "Culture of Hematopoïetic cells, R.I. Freshney, I.B. Pragnell, M.G. Freshney ed, Wiley-Liss" pp. 205-221. Le titre du chapitre est "Purification and in vivo assay of early human hematopoïetic progenitors" J. Hatzfeld, P. Batard, A.A. Cardoso, M.L. Li, A. Hatzfeld.

Conformément à l'invention, on peut évaluer la maturité ou l'immaturité des cellules souches après le transfert de gènes. Cette évaluation se fait comme indiqué ci-dessus, à partir des colonies les plus grosses pour lesquelles on vérifie simultanément à la susdite évaluation d'immaturité, qu'il y a bien eu transfert de gènes, comme indiqué ci après dans l'exemple 1.

L'invention concerne notamment un procédé de transfert de gènes dans la cellule souche hématopoïétique dans lequel on évalue l'immaturité des cellules souches avant le transfert de gène, et avantageusement après le transfert de gènes.

Ce procédé peut comprendre les étapes suivantes :
1- Evaluation des cellules hématopoïétiques à greffer pour s'assurer qu'elles contiennent des cellules immatures désignées ci-dessus par HPP-Q, élément du compartiment de la cellule souche. Il est en effet inutile de procéder à un transfert de gènes si les cellules utilisées ne sont pas les bonnes. Par le procédé d'évaluation de l'immaturité des cellules souches, on peut comparer une culture dans un milieu semi-solide ou liquide, sans anti-inhibiteur du cycle cellulaire avec la même culture en présence d'un anti-inhibiteur. La différence du nombre de colonies doit mettre en évidence l'apparition de nouvelles colonies plus grosses que les précédentes, dérivées de cellules HPP-Q.
2- Préstimulation des cellules hématopoïétiques avec un milieu contenant un activateur du cycle cellulaire (10 heures pour un anti-TGF-β), pour sortir les cellules hématopoïétiques de la phase de repos, c'est-à-dire pour les activer.
3- Transfert de gènes avec un surnageant viral ou en coculture avec des cellules productrices de virus dans un milieu contenant un anti-interferon et/ou un anti-TGF-β.
4- Après le transfert de gènes, les cellules hématopoïétiques activées devraient pouvoir être remises au repos (phase Go) dans un milieu de désactivation contenant un inhibiteur de cette cellule (TGF-β dans le cas de la cellule souche hématopoïétique).
5- Alternativement, les cellules activées ayant subi un transfert de gènes ou non, peuvent être amplifiées pour être réinjectées in vivo. Ceci peut être réalisé avec une sous-population des cellules à réinjecter pour empêcher l'aplasie qui précède généralement la reprise d'une greffe.
6- Avant ou après réinfusion des cellules, il est nécessaire de faire un test HPP-Q, suivi d'une coloration in situ ou d'une RT-PCR (reverse transcriptase-polymerase chain reaction = transcriptase inverse- amplification en chaîne par polymérase) des colonies obtenues pour évaluer le transfert de gènes.

Comme milieu de préstimulation et avant le transfert de gènes, on peut utiliser des milieux de culture liquide tels que ceux décrits dans l'article de Cardoso et al. (Proc. Natl. Acad. Sci. USA, Vol. 90, pp. 8707-8711, September 1993).

Le procédé de l'invention permet d'évaluer le nombre de cellules primitives au repos (HPP-Q) avant le transfert de gènes, pour déterminer la richesse en cellules souches, ou après le transfert de gènes, pour déterminer l'efficacité du transfert de gènes dans le compartiment de la cellule souche.

### Description des figures.

La figure 1 représente une colonie CFU-GEMM exprimant de façon stable le gène nls-Lac-Z mis en évidence par la coloration X Gal.

La figure 2 représente à plus fort grossissement, un groupe de cellules érythroïdes de la colonie CFU-GEMM avec une expression du gène nls-lacZ homogène dans toutes les cellules.

La figure 3 montre la localisation nucléaire de la β-galactosidase dans les macrophages et l'absence de coloration non spécifique dans le cytoplasme.

### EXEMPLE 1: TRANSFERT DU GENE NLS-LACZ DANS DES CELLULES SOUCHES HEMATOPOÏETIQUES.

### Matériel et méthodes.

### Facteurs de croissance hématopoïétique et antisérum.

L'interleukine 3 (IL3) humaine recombinante, l'interleukine 6 (IL6) humaine recombinante, le GM-CSF (granulocyte macrophage colony-stimulating factor) humain recombinant et le G-CSF (granulocyte colony-stimulating factor) humain recombinant sont fournis par Genetics Institute (Cambridge, MA). L'érythropoïétine humaine recombinante est fournie par AMGEN (Thousand Oaks, CA), et *le* SF (Steel factor) humain recombinant provient d'IMMUNEX (Seattle, WA). Les activités spécifiques de ces cytokines sont respectivement: 2,3 x 10⁶ U/mg, 4 x 10⁶ U/mg, 9.3 x 10⁶ U/mg, 7 x 10⁴ U/mg, 4.98 x 10⁵ U/mg et 10⁶ U/mg. L'antisérum bloquant de dinde, anti-TGF-β, est un don du Dr. A.B. Roberts et du MB Sporn (Danielpour, D., Dart, L. L., Flanders, K. C., Roberts, A. B., & Sporn, M. B. (1989) J. Cell. Physiol. 138, 79-86): 1 µl peut neutraliser 4 ng de TGF-β. Il est utilisé à la concentration de 4,2 µl/ml.

### Purification de cellules CD34+

Par cellules CD34+, on désigne des cellules portant l'antigène membranaire CD34 et comprenant une sous population qui représente le compartiment de la cellule souche.

### Récupération de sang de cordon ombilical.

Des échantillons de sang de cordon ombilical humains sont récupérés à partir de cordon ombilical/placenta immédiatement après la naissance, tandis que le placenta reste *in situ* (Broxmeyer, H. E., Douglas, G. W., Hangoc, G., Cooper, S., Bard, J., English, D., Arny, M., Thomas, L., & Boyse, E. A. (1989) Proc. Natl. Acad. Sci. USA **86,** 3828-3832; Gluckman, E., Devergie, A., Thierry, D., Eperou-Bourdeau, H., Traineau, R., Gerrota, J., Brossard, Y., Van Nifterik, J., & Benbunan, M. (1992) Bone Marrow Transplant. 9, 114-117).

### Purification de cellules SBA-CD34+

Les cellules mononucléaires sont obtenues comme décrit précédemment (Hatzfeld, J., Li, M.-L., Brown, E. L., Sookdeo, H., Levesque, J.-P., O'Toole, T., Gurney, C., Clarck, S.C., & Hatzfeld, A. (1991) J. Exp. Med. 174, 925-929). Très brièvement après l'isolation de cellules mononucléaires sur du Ficoll Hypaque, les cellules SBA- (cellules ne se fixant pas à l'agglutinine de soja) sont purifiées en éliminant les cellules restées attachées à l'agglutinine de soja immobilisée de façon covalente en utilisant la procédure AIS, (procédure mise au point par la firme Applied Immune Sciences). (BSA CELLector Flasks, Applied Immune Sciences Inc. Santa Clara, CA). La sélection positive de CD34+ est obtenue avec des flacons de culture recouverts d'anticorps monoclonaux CD34 ICH3 (CD34 CELLector Flasks, AIS), lavées un nombre suffisant de fois (jusqu'à 10 fois) avec une solution saline tamponnée de phosphate Dulbecco, contenant 1 mM d'EDTA pour assurer l'élimination des cellules non attachées et faiblement attachées (CD34^{+faible}) comme observé sur le microscope inversé (Hatzfeld J., Batard P., Cardoso A.A., Li M.-L., et Hatzfeld A. Purification and *in vitro* assay of early human hematopoietic progenitors. 205-221. Dans Culture of Hematopoiectic Cells. Freshney R.I., Pragnell I.B., Freshney M.G., 1994).

Cette procédure donne de façon routinière une pureté de 95% (± 3%) de cellules CD34. Les cellules isolées CD34+SBA- sont ensuite détachées par une incubation de 2 heures à 37°C dans du milieu d'Iscove modifié par Dulbecco (IMDM) additionné de sérum de veau foetal (FCS) à 10% ou dans du milieu Stem GEM* du CNRS (milieu sans sérum). Le détachement des cellules doit être complet, étant donné que les cellules qui expriment une quantité élevée d'antigènes CD34 représentent les progéniteurs les plus immatures et sont les plus difficiles à détacher.

### Composition des milieux

Les milieux de préstimulation et de transfert sont des milieux Stem GEM* avec ou sans sérum (CNRS Centre National de la Recherche Scientifique UPR 9044 Villejuif France).

### Procédé de transduction

La lignée de cellules NB16, une lignée de cellules (Danos O., et Mulligan R.C.: "Safe and efficient generation of recombinant retroviruses with amphotropic and ecotropic host ranges". Proc. Natl. Acad. Sci., USA **85:** 6460-6464, 1988) qui exprime la β-galactosidase d'*E. coli* (Strair, R. K., Towle. M., & Smith, B. R. (1990) Nucleic Acids Res. **18,** 47-4762) est fournie par J.M. Heard et O. Danos. Ces cellules sont maintenues en culture dans du milieu DMEM additionné de 10% sérum de veau nouveau né enrichi en fer (Hyclone). Cette lignée cellulaire produit un surnageant avec un titre viral habituel de 1 à 5.10⁵ unités/ml. Les cellules CD34+ sont préstimulées pendant des temps variés (50 000 cellules de CD34+ dans 0.5 ml). Elles sont ensuite mises en co-culture pendant 40 h sur des cellules NB16 à 40% de confluence dans du milieu StemGEM* (CNRS) additionné de polybrène à 8 µl/ml à 37°C avec 5% de CO₂ dans une atmosphère saturée en humidité. Les cellules de CD34+ sont ensuite doucement détachées, concentrées et testées pour des essais clonogéniques.

### Essais clonogéniques dans la méthylcellulose.

Les cellules CD34+ sont cultivées à raison de 300-400 cellules/ml selon une modification d'un test de colonie mixte de Fauser et Messner (Hatzfeld, J., Li, M.-L., Brown, E. L., Sookdeo, H., Levesque, J.-P., O'Toole, T., Gurney, C., Clarck, S.C., & Hatzfeld, A. (1991) J. Exp. Med. 174, 925-929; Fauser, A. A., & Messner, H. A. (1979) Blood 53, 1023). Toutes les cultures sont faites au moins en double.

### Coloration des colonies transduites.

Après une incubation de 14 à 21 jours à 37°C dans une atmosphère humidifiée saturée, les colonies sont comptées et classifiées comme décrit de façon antérieure (Zhou, Y.-Q., Stanley, E. R., Clark, S. C., Hatzfeld, J., Levesque, J.-P., Federici, C., Watt, S. M., & Hatzfeld, A. (1988) Blood 72, 1870-1874). Elles sont ensuite colorées par la même méthode histochimique que celle utilisée pour évaluer le titre d'un surnageant, avec X-Gal comme substrat (MacGregor, G. R., Nolan, G. P., Fiering, S., Roederer, M., & Herzenberg, L. A. (1991) Methods Mol. Biol. 7, 217-235) à l'exception du fait que la solution de coloration (1 ml/boîte de Petri) est deux fois plus concentrée que pour les cellules adhérentes. La réaction de coloration est effectuée toute la nuit.

### Analyse statistique.

La signification de différences entre les groupes de traitement est déterminée en utilisant le test *t* de Student.

### Résultats.

L'efficacité d'infection de nls-lacZ dépend d'une période de préstimulation de 10 h avec l'anti-TGF-β.

Les cellules SBA-CD34⁺⁺⁺ (cellules CD34 les plus immatures) sont purifiées avec une récupération élevée de cellules du compartiment de la cellule souche dont font partie les CFU-GEMM (colony-forming unit - granulocyte/erythrocyte /macrophage /megacaryocyte ) ayant un potentiel de prolifération élevé (supérieur à 10⁵ cellules) comme décrit dans Matériel et méthodes. Ces cellules sont préincubées pendant des temps variés avec ou sans sérum anti-TGF-β comme détaillé dans le tableau 1 avant la co-culture avec des cellules NB16 pour infection par des vecteurs rétroviraux. Lorsque ces cellules sont préstimulées pendant 10 h avec uniquement des cytokines et sans anti-TGF-β avant la transduction, l'efficacité de transfert augmente de 1,1 à 23,8%. Cette augmentation double de façon significative en présence de sérum anti-TGF-β, s'élevant de 23,8 à 47,3%. L'efficacité d'activation de l'anti-TGF-β disparaît pendant des temps de préincubation plus longs. Des périodes de préstimulation de 20 ou 48 h, ou plus longues (non représentées), avec ou sans sérum anti-TGF-β fournissent des résultats similaires comme ceux observés après 10 h sans sérum anti-TGF-β.

### Effet d'anti-TGF-β sur l'efficacité de transfert de nls-lacZ dans les différents types de progéniteurs.

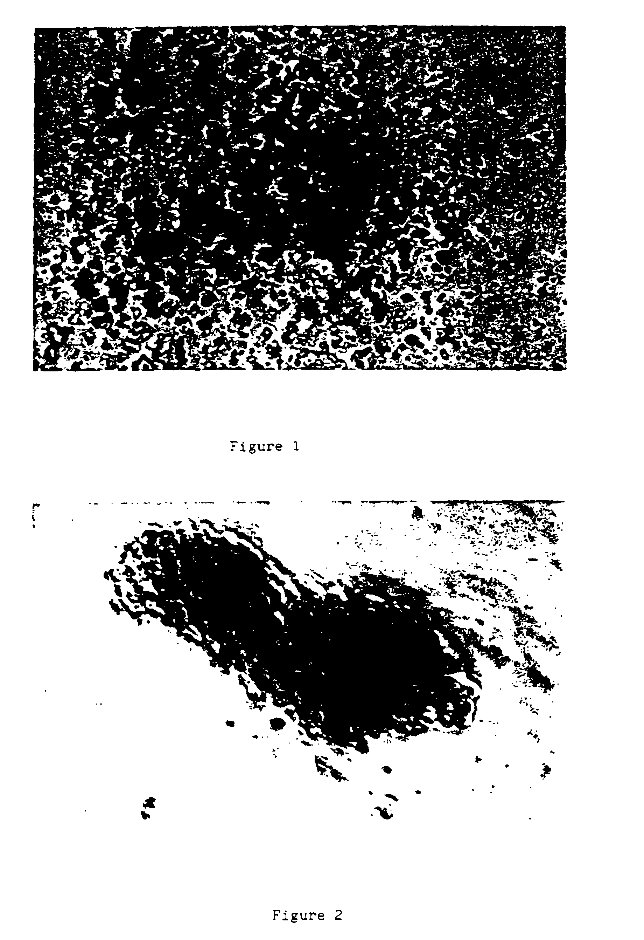

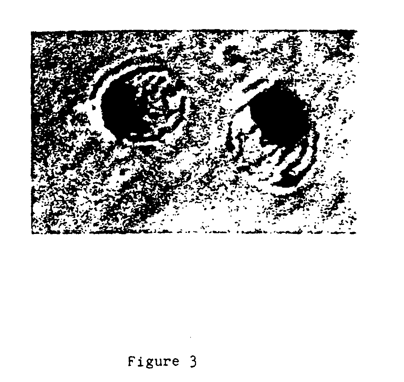

Etant donné qu'une préstimulation de 10 h en présence de sérum anti-TGF-β donne une augmentation significative du nombre total de colonies transduites, on a utilisé cette condition de préstimulation dans les expériences suivantes. Le tableau 2 détaille les types variés de progéniteurs qui sont transduits avec cette méthode et l'efficacité relative d'expression pour chacun d'eux. L'efficacité d'expression la plus élevée est obtenue avec les progéniteurs les plus immatures qui sont les colonies mixtes provenant de CFU-GEMM. Ces progéniteurs donnent des colonies contenant 1 à 2 x 10⁵ cellules. La figure 1 montre une telle colonie. La figure 2 représente des cellules érythroïdes avec une expression du gène nls-Lac-Z homogène dans toutes les cellules. La figure 3 détaille de façon plus précise la localisation nucléaire de β-galactosidase dans les macrophages et prouve l'absence de coloration non spécifique dans le cytoplasme. La préstimulation par l'anti-TGF-β augmente l'efficacité de transfert de 54,5 à 95% dans les CFU-GEMM (colony-forming unit - granulocyte/ erythrocyte/macrophage/megacaryocyte), de 43,8 à 66,2% dans les BFU-E (burst-forming unit - erythroide), de 3,5 à 13,5% dans les CFU-GM (colony-forming unit - granulocyte /macrophage), de 0 à 9,3% dans CFU-G (colony-forming unit - granulocyte) et de 8,3 à 22,2% dans les CFU-M (colony-forming unit - macrophage). (L'efficacité de transfert est augmentée par rapport aux conditions de transfection de CFU-GEMM sans TGF-β).

La préstimulation par l'anti-TGF-β augmente la stabilité de l'expression du gène pendant le développement des CFU-GEMM.

Etant donné que les CFU-GEMM représentent une sous-population du compartiment de la cellule souche qui prend part à la reconstitution hématopoïétique à long terme (Lu, L., Xiao, M., Shen, R.-N., Grisby, S., & Broxmeyer, H. E. (1993) Blood **81,** 41-48), on a analysé la stabilité de l'expression du gène pendant le développement des CFU-GEMM en colonies mixtes. Dans le tableau 3, les CFU-GEMM sont classés selon le pourcentage de cellules exprimant nls-lacZ, à l'intérieur de chacune des colonies. La préstimulation par du sérum anti-TGF-β augmente le nombre de colonies contenant de 90 à 100% de cellules exprimant le gène nls-lacZ. Ce type de CFU-GEMM ne se développe pas dans les cultures de cellules qui ne sont pas préstimulées par un sérum anti-TGF-β. Au contraire, cette sous-population représente 41,2% de toutes les CFU-GEMM transduites après la préstimulation par du sérum anti-TGF-β.

On a déterminé si la taille des colonies est modifiée par le transfert de gènes. On observe que le nombre de cellules n'est pas différent de façon significative dans les colonies transduites par nls-lacZ par rapport à celles qui ne le sont pas.

**Tableau 1.**

| **Le sérum anti-TGF-β ajouté pendant une préstimulation de** **10 h augmente l'efficacité de transfert de gène.** | | | | |
|---|---|---|---|---|
| Heures | Antisérum anti-TGF-β | Nombre total de colonies | Colonies transfectées | % de transfert |
| 0 | - | 103 (± 16) | 1 (± 0) | 1.1 (± .05) |
| | + | 94 (± 18) | 2 (± 2) | 1.54 (± .4) |
| | | *df = 2; p = 0.330* | *df = 2; p = 0.028* | *df = 2; p = 0.128* |
| 10 | - | 74 (± 19) | 15 (± 5) | 23.8 (± 9.0) |
| | + | 125 (± 15) | 51 (± 10) | 24.8 (± 2.09) |
| | | *df = 3; p = 0.001* | *df = 3; p = 0.0004* | *df = 3; p =0.034* |
| 20 | - | 110 (±7) | 24 (± 5) | 23.5(± 1.66) |
| | + | 140 (± 4) | 35 (± 4) | 24.8 (± 2.2) |
| | | *df = 2; p = 0.015* | *df = 2; p = 0.085* | *df = 2; p = 0.394* |
| 48 | - | 91 (± 15) | 20 (± 6) | 21.9 (± 3.7) |
| | + | 113 (± 10) | 21 (± 4) | 18.7 (± 2.2) |
| | | *df = 2; p = 0.138* | *df = 2; p = 0.440* | *df = 2; p = 0.394* |

Le nombre de colonies est obtenu avec 10³ cellules/ml mises en culture dans de la méthylcellulose comme décrit dans Matériel et Méthodes.

**Tableau 2.**

| **Effet du sérum anti-TGF-β sur l'efficacité de transfert dans** **différents types de progéniteurs hématopoïétiques.** | | | |
|---|---|---|---|
| Progéniteurs | | % de transfert sans préstimulation par l'anti-TGF-β | % de transfert avec préstimulation avec l'anti-TGF-β |
| CFU-GEMM | Nombre total de colonies | 11 | 21 |
| | Colonies transfectées | 6 | 20 |
| | % de transfert | 54.5 | 95 |
| BFU-E | Nombre total de colonies | 67 | 83 |
| | Colonies transfectées | 19 | 55 |
| | % de transfert | 43.8 | 66.2 |
| CFU-GM | Nombre total de colonies | 28 | 38 |
| | Colonies transfectées | 1 | 5 |
| | % de transfert | 3.5 | 13.5 |
| CFU-G | Nombre total de colonies | 21 | 43 |
| | Colonies transfectées | 0 | 4 |
| | % de transfert | 0 | 9.3 |
| CFU-M | Nombre total de colonies | 12 | 18 |
| | Colonies transfectées | 1 | 4 |
| | % de transfert | 8.3 | 22.2 |
| CFU-GEMM : colony-forming unit - granulocyte/erythrocyte/macrophage/mégacaryocyte; | | | |
| BFU-E : burst-forming unit - erythroïd; | | | |
| CFU-GM : colony-forming unit - granulocyte/macrophage; | | | |
| CFU-G : colony-forming unit - granulocyte; | | | |
| CFU-M : colony-forming unit - macrophage. | | | |

Ceci est le résultat d'une expérience parmi 3 expériences similaires.

**Tableau 3.**

| **Distribution des CFU-GEMM transfectées selon la stabilité** **de l'expression du gène nls-Lac-Z pendant le développement de la colonie.** | | | | |
|---|---|---|---|---|
| | Sans sérum anti-TGF-β | | Avec sérum anti-TGF-β | |
| % de cellules exprimant le gène nls-Lac-Z dans chaque colonie mixte | % de colonies mixtes | Nombre de cellules par colonie mixte | % de colonies mixtes | Nombre de cellules par colonie mixte |
| 90-100 | 0 | 118 706 (±16 300) | 41,2 | 152 776 (± 18 336) |
| 40-90 | 66.6 | 121 766 (±10 821) | 35,3 | 156 456 (± 19 069) |
| 0-40 | 33.3 | 122 313 (± 13 590) | 23,5 | 169 963 (± 16 919) |

Les résultats représentent la moyenne (± SD) de 3 expériences indépendantes.

**Tableau 4.**

| **Effet de l'antisérum anti-TGF-β et/ou de l'anti-interféron (IFN) sur le** **titre du surnageant produit par les cellules NB16.** | | | |
|---|---|---|---|
| Surnageant | Contrôle | Antisérum anti-IFN | Antisérum anti-TGF-β |
| CFU/10⁻⁵ | 6 (± 4.4) | 65.8 (± 68.7) | 38.2 (± 42.02) |
| nombre d'expériences | (n=6) | (n=6) | (n=4) |

Des cellules NB16 ont été cultivées en absence ou en présence d'anti-TGF-β ou d'anti-interféron.

Les surnageants sont ensuite ajoutés à des cultures de cellules 3T3 non confluantes. Lorsque les cellules sont confluantes, elles sont colorées par le X-Gal et les foyers viraux sont comptés et le titre est exprimé par le nombre de foyers viraux multiplié par l'inverse de la dilution du surnageant de départ (CFU : Colony Forming Unit).

L'addition d'anti-TGF-β ou d'anti-interféron pendant la coculture peut augmenter le pourcentage de transfert (résultat non montré) dans les cellules hématopoïétiques par un effet direct sur la lignée productrice. Le tableau 4 montre, en effet, que lorsqu'on ajoute aux cellules NB16 de l'anti-TGF-β ou un agent viral comme un anti-interféron, on augmente le titre du surnageant. En effet, le surnageant obtenu après traitement par l'anti-interféron augmente de 11 fois le nombre de foyers d'infection et avec l'anti-TGF-β, il augmente de 6 fois.

### EXEMPLE 2

On donne ci-après les résultats obtenus dans des essais comparatifs de préstimulation avec ou sans anti-inhibiteur du cycle cellulaire.

But de l'expérience :

On souhaite sortir de la phase de repos des progéniteurs primitifs qui seraient sous le contrôle d'un TGFβ autocrine. Cette sortie est obtenue après un temps de stimulation court (10 heures) puis le phénomène est mis en évidence par mise en culture semi-solide des progéniteurs activés et lecture du nombre de colonies mixtes après 21 jours de culture (Fauser A.A. et Messner H.A. (1979), Identification of megakaryocytes, macrophages, and eosinophils in colonies of human bone marrow containing neutrophilic granulocytes and erythroblasts. Blood, **53**:1023-1027).

### Procotole expérimental :

Purification des cellules CD34+ de sang de cordon ombilical.

Les cellules sont incubées dans 2 milieux de base différents :
A : milieu GTL
B : milieu liquide standard avec 10% de sérum de veau foetal.

La composition du milieu GTL est décrite dans l'article suivant "Specific role of lipids, transferrin and insulin in defined media for cells of the human hematopoïetic and immune systems" J. Hatzfeld, A. Hatzfeld, J. Maigné, M. Sasportes, R. Willis and D.B. Mc Clure in Cold Spring Harbor Conferences on Cell proliferation vol. 9, Growth of Cells in hormonally defined media, 1982, pp. 703-710.

La composition du milieu GTL est la suivante:
IMDM : milieu de base
+ 1 mg/ml serum albumine bovine délipidée reconstituée avec 5 µg/ml acide oléique, 5 µg/ml acide linoléique, 1 µg/ml acide palmitique,
+ transferrine 5 µg/ml ("Specific role of lipids, transferrin and insulin in defined media for cells of the human hematopoïetic and immune systems" J. Hatzfeld, A. Hatzfeld, J. Maigné, M. Sasportes, R. Willis and D.B. Mc Clure in Cold Spring Harbor Conferences on Cell proliferation vol. 9, Growth of Cells in hormonally defined media, 1982, pp. 703-710).

La composition du milieu standard est la suivante:
- IMDM +: 10% sérum de veau foetal
1 mg/ml serum albumine bovine
2 mM glutamine
10 µg/ml transferrine
4x10⁻⁵ M β-mercaptoéthanol.

A ces deux milieux de base sont ajoutées 6 au moins des cytokines suivantes : IL1, IL2, IL3, IL4, ILS, IL6, IL7, IL11, GCSF, SF, EPO, GM-CSF, FL, TPO, FGF, insuline ou des synthokines activant les récepteurs correspondant à ces cytokines.

Dans chaque groupe de milieux A et B , on teste les conditions suivantes:
1) contrôle : rien d'autre n'est ajouté,
2) addition d'un anticorps anti-TGFβ provenant de Stem GEM* CNRS, 7 rue Guy Môquet, 94801 Villejuif, FRANCE.

Les cellules sont mises à l'étuve pendant 10 heures à 37°C, 5% CO2; 1000 cellules dans 0,4 ml de milieu.

A la fin de la période de préstimulation, on ajoute 2,1 ml de milieu semi-solide aux cellules. On met en incubation pendant 21 jours à 37°C, 5% CO.

### Résultats :

Chaque chiffre représente la moyenne de deux boîtes. Ne sont présentés que les comptages des colonies mixtes (colonies constituées de cellules rouges + un ou plusieurs types de lignages de cellules blanches) qui proviennent des cellules les plus immatures.

| | Exp A | Exp B | Exp C |
|---|---|---|---|
| Contrôle (1) | 8 | 6 | 1 |
| | | | |
| En présence d'anticorps anti-TGFβ (2) | 15 | 12 | 4 |

### EXEMPLE 3:

Par ailleurs, on a vérifié que des combinaisons de facteurs plus élaborées que celles déjà utilisées n'ont pas un effet aussi efficace que l'anti-TGFβ.

On a comparé de nombreuses combinaisons de cytokines plus élaborées que les précédentes dans des cultures avec ou sans anti-TGF-β. On observe que certaines combinaisons de cytokines peuvent avoir un effet semblable à l'effet de l'anti-TGF-β sur les colonies qui se développent rapidement et donc proviennent de progéniteurs tardifs déjà activés. Par contre, l'effet de l'anti-TGF-β ne peut être remplacé par aucune combinaison de cytokines pour le développement de colonies sur 18 jours.

Par ailleurs, s'il était connu que les cytokines induisent le récepteur amphotropique des rétrovirus utilisés pour un transfert de gènes, on a montré, grâce à l'invention, que l'anti-TGFβ augmente l'expression des récepteurs de cytokines, donc leur action, donc l'expression des récepteurs amphotropes, donc le transfert de gènes. L'augmentation de l'expression des récepteurs de cytokines pour l'anti TGF-β est surtout nette sur les cellules au repos.

### EXEMPLE 4:

Expérience en condition de cultures unicellulaires (cellules CD34+CD38 faibles) comparant l'effet de FL/bFGF (Ligand Flik/facteur de croissance fibroblastique basique) et anticorps anti-TGF-β1(provenant de Stem GEM*) dans le milieu suivant: milieu B: sérum /IL3/IL6/SF/GCSF) à J0, et milieu B+GMCSF+EPO à J12.

On a utilisé quatre conditions de milieu de culture :
1. milieu standard avec 24% sérum de veau foetal et 4 cytokines (IL3, IL6, SF, GCSF),
2. milieu standard avec 10% sérum de veau foetal et 4 cytokines (IL3, IL6, SF, GCSF) + FL/bFGF,
3. milieu standard + anticorps anti-TGFβ (provenant de Stem GEM*).
4. milieu standard + FL/bFGF + anticorps anti-TGF-β (provenant de Stem GEM*).

### Résultats :

Les quatre conditions donnent les résultats suivants :
- Pour la mise en cycle des cellules :
   1. 50% des cellules donnent des clones de plus de 100 cellules,
   2. 75% des cellules donnent des clones de plus de 100 cellules,
   3. 75% des cellules donnent des clones de plus de 100 cellules,
   4. 75% des cellules donnent des clones de plus de 100 cellules,
      Il n'y a donc pas d'effet additif dans la combinaison 4).
      1. Il semblerait que ce soit la même population qui soit en cause.
- Pour la prolifération, les conditions 2 et 3 donnent les résultats suivants :
   2. 15-20% des clones contiennent plus de 10⁴ cellules,
   3. 50% des clones contiennent plus de 10⁴ cellules.

Donc l'anticorps anti-TGF-β est plus efficace que le FL/bFGF.
- Sur la nature des cellules obtenues :
   . avec la combinaison 2, on obtient surtout des G et des M (cellules plus matures) c'est-à-dire des granulocytes et des monocytes,
   . avec les combinaisons 3 et 4, on obtient surtout des cellules rouges et des mixtes (c'est-à-dire mélange de cellules rouges et de cellules blanches).

Donc l'anticorps induit la mise en cycle de cellules plus précoces puisque c'est seulement avec l'anticorps anti-TGF-β qu'on stimule les voies érythroïdes ou mixtes.

L'ensemble de ces résultats permet de dégager les avantages du traitement court par l'anti TGF-β selon l'invention.

Toutes les publications concernant le transfert de gènes insistent sur les deux paramètres importants d'un transfert efficace : un surnageant à titre viral élevé et la mise en cycle de la cellule à infecter. C'est la raison pour laquelle on stimule les cellules souches avec des cytokines. Dans les publications, la stimulation a lieu pendant plus de 72 heures et concerne des cellules déjà en cycle. Au bout de 72 heures, beaucoup de ces cellules ont déjà commencé à se différencier. Les cellules qui sont infectées sont donc beaucoup moins immatures qu'au départ. Avec l'anti-TGF-β, et conformément au procédé de l'invention, les cellules sont stimulées un temps très court et sont des cellules initialement au repos. Elles n'ont eu que le temps d'entrer en cycle sans se différencier ou peu. Le procédé de l'invention permet de les remettre en phase de repos immédiatement après le transfert de gènes grâce à un court traitement par du TGF-β. Par conséquent, les cellules qui sont transfectées sont plus immatures, ce qui est le but recherché en thérapie génique utilisant la cellules souche hematopoïétique. Ce procédé est applicable à d'autres types de cellules en utilisant éventuellement d'autres anti-inhibiteurs du cycle cellulaire (anti Rb : anti-gène de susceptibilité au rétinoblastome, anti INK : anti-inhibiteur de kinase, etc).

Ces anti-inhibiteurs peuvent être des oligonucléotides anti-sens, des anticorps bloquants, des inhibiteurs naturels ou de synthèse.

## Revendications

1. Utilisation de moyens de blocage d'au moins un inhibiteur du cycle cellulaire pendant une durée n'excédant pas 72 h, et n'excédant pas de préférence 36 h, et notamment inférieure à environ 20 h, et de préférence d'environ 1 à 15 h, et de préférence d'environ 1 à 10 h, dans une culture de cellules souches hématopoïétiques, afin de libérer les cellules souches hématopoïétiques de leur état de repos.

2. Utilisation selon la revendication 1, de moyens de blocage d'au moins un inhibiteur du cycle cellulaire dans un test de comparaison, d'une part, entre une culture de cellules souches hématopoïétiques dans un milieu contenant les sus-dits moyens de blocage d'au moins un inhibiteur du cycle cellulaire et, d'autre part, entre une même culture de cellules souches hématopoïétiques que celle mentionnée ci-dessus dans un milieu ne contenant pas de sus-dit moyen de blocage d'au moins un inhibiteur du cycle cellulaire, afin de déterminer le degré de maturité des sus-dites cellules souches hématopoïétiques.

3. Utilisation selon la revendication 1, de moyens de blocage d'au moins un inhibiteur du cycle cellulaire dans une culture de cellules souches hématopoïétiques, afin de libérer les cellules souches hématopoïétiques de leur état de repos, suivie d'une étape de transfert de gènes dans les sus-dites cellules souches hématopoïétiques.

4. Utilisation selon l'une des revendications 1 à 3 dans laquelle les cellules souches sont des cellules hématopoïétiques et les moyens de blocage d'au moins un inhibiteur du cycle cellulaire sont constitués par de l'anti TGF-β.

5. Cellules souches hématopoïétiques, contenant tout ou partie d'un gène hétérologue dans leur génome, susceptibles d'être obtenues selon le procédé comprenant les étapes suivantes:
- la préstimulation de cellules souches hématopoïétiques au repos dans un milieu contenant:
* des moyens de blocage directs ou indirects d'au moins un inhibiteur du cycle cellulaire des dites cellules souches hématopoïétiques pendant une durée suffisante pour libérer les cellules souches hématopoïétiques de leur état de repos, et n'excédant pas 72 h, et n'excédant pas de préférence 36 h, et notamment inférieure à environ 20 h, et de préférence d'environ 1 h à 15 h, et de préférence d'environ 1 h à 10 h, et
* des substances, notamment des cytokines, pour permettre l'activation d'un cycle cellulaire et/ou ultérieurement la traversée d'au moins un cycle cellulaire,
- l'étape de transfert par la mise en présence des cellules souches hématopoïétiques obtenues à l'étape précédente avec tout ou partie d'un gène hétérologue, dans un milieu permettant le transfert de tout ou partie du gène hétérologue dans le génome des cellules souches hématopoïétiques, pour obtenir des cellules souches hématopoïétiques comportant tout ou partie d'un gène hétérologue,
- la récupération éventuelle des cellules souches hématopoïétiques obtenues à l'étape précédente.

6. Cellules souches hématopoïétiques selon la revendication 5 contenant dans leur génome tout ou partie d'un gène hétérologue, et ayant un potentiel de multiplication égal ou supérieur à 10⁶ et un potentiel de différenciation susceptible de couvrir tous les lignages hématopoïétiques.

7. Cellules souches hématopoïétiques selon l'une des revendications 5 ou 6, comportant, à l'intérieur d'elles mêmes ou sur leur surface, des moyens de blocage de tout inhibiteur de leur cycle cellulaire.

8. Cellules, selon l'une quelconque des revendications 5 à 7, à l'état de repos.

9. Cellules souches hématopoïétiques selon l'une des revendications 5 à 8, dans lesquelles les moyens de blocage sont constitués par ceux choisis parmi: les anti-inhibiteurs du cycle cellulaire, par exemple des agents bloquant les gènes suppresseurs de tumeurs, des antagonistes du TGF-β tels que des antisens du TGF-β, des anticorps neutralisants le TGF-β, des récepteurs solubles du TGF-β ou des inhibiteurs naturels ou de synthèse du TGF-β, ou des inhibiteurs de la voie de synthèse du TGFβ.

10. Cellules souches hématopoïétiques selon l'une des revendications 5 à 9, dans lesquelles tout ou partie du gène hétérologue peut être choisi parmi les gènes suivants: marqueurs: Neo (gène de résistance à la néomycine), CD2 (antigène leucocytaire), nls-Lac-Z, ou des gènes corrigeant une hématopathie ou tout autre pathologie telle que ADA (gel de l'adénosine déaminase), ALDP (gène corrigeant adrénoleucodystrophie), TK (gène suicide permettant de tuer des cellules en présence d'acyclovir sous le contrôle de promoteurs viraux).

11. Procédé de transfert de tout ou partie d'un gène hétérologue dans le génome de cellules souches hématopoïétiques activées à partir d'un état de repos, **caractérisé en ce qu'**il comprend les étapes suivantes:
- la préstimulation des cellules souches hématopoïétiques au repos dans un milieu contenant:
* des moyens de blocage directs ou indirects d'au moins un inhibiteur du cycle cellulaire des dites cellules souches hématopoïétiques pendant une durée suffisante pour libérer les cellules, notamment les cellules souches hématopoïétiques de leur état de repos, et n'excédant pas 72 h, et n'excédant pas de préférence 36 h, et notamment inférieure à environ 20 h, et de préférence d'environ 1 h à 15 h, et de préférence d'environ 1 h à 10 h, et
* des substances, notamment des cytokines, pour permettre l'activation d'un cycle cellulaire et/ou ultérieurement la traversée d'au moins un cycle cellulaire,
- l'étape de transfert par la mise en présence des cellules souches hématopoïétiques obtenues à l'étape précédente avec tout ou partie d'un gène hétérologue, dans un milieu permettant le transfert de tout ou partie du gène hétérologue dans le génome des cellules souches hématopoïétiques, pour obtenir des cellules souches hématopoïétiques comportant tout ou partie d'un gène hétérologue,
- la récupération éventuelle des cellules souches hématopoïétiques obtenues à l'étape précédente.

12. Procédé selon la revendication 11, dans lequel l'étape de transfert de tout ou partie du gène hétérologue se fait avec une coculture de cellules souches hématopoïétiques, préstimulées selon la première étape de la revendication 11, et de cellules produisant un vecteur, tel qu'un vecteur viral ou un rétrovirus, contenant tout ou partie du gène à transférer.

13. Procédé selon la revendication 11, dans lequel l'étape de transfert de tout ou partie du gène hétérologue se fait en mettant en présence des cellules souches hématopoïétiques, préstimulées selon la première étape de la revendication 11, et du surnageant de cellules contenant un vecteur, tel qu'un vecteur viral ou un rétrovirus comprenant tout ou partie du gène à transférer.

14. Procédé selon l'une des revendications 11 à 13, dans lequel tout ou partie du gène à transférer provient d'un vecteur, tel qu'un vecteur viral ou un rétrovirus produit par une culture productrice à laquelle est additionné un agent viral, tel qu'un anti-TGF-β, un anti-interféron ou tout agent stabilisateur de vecteur.

15. Procédé selon l'une des revendications 11 à 14, dans lequel le milieu de préstimulation comprend: IMDM (Iscove Dubelcco Modified Medium), (Stem GEM*) correspondant à un milieu commercialisé par le C.N.R.S.), des cytokines, des antagonistes du TGF-β tels que des oligonucléotides antisens du TGF-β, des anticorps neutralisants le TGF-β, des récepteurs solubles du TGF-β ou des inhibiteurs naturels ou de synthèse du TGF-β, des anticorps neutralisants le TGF-β, des récepteurs solubles du TGF-β ou des inhibiteurs naturels ou de synthèse du TGF-β, des combinaisons de cytokines susceptibles de bloquer dans un espace de temps court (inférieur à 72 h, de préférence inférieur à 10 h), les inhibiteurs du cycle cellulaire des cellules souches quiescentes.

16. Procédé selon l'une des revendications 11 à 15, dans lequel les **substances permettant l'activation d'un cycle cellulaire et/ou ultérieurement la traversée d'au moins un cycle cellulaire sont choisies parmi une combinaison d'au moins 4 substances choisies parmi les suivantes:** IL1, IL2, IL3, IL4, ILS, IL6, IL7, IL11, GM-CSF (granulocyte-monocyte colony stimulating factor), G-CSF, facteur Steel (SF), ligand de FLT3 (FL), érythropoïétine, TNF, LIF, thrombopoïétine et insuline ou synthokines de synthèse.

17. Procédé selon l'une des revendications 11 à 16, dans lequel le milieu permettant le transfert de tout ou partie du gène hétérologue est constitué par, ou comprend: DMEM, et éventuellement IMDM, des cytokines, et éventuellement un antagoniste du TGF-β, des anti-interférons, ou tout autre agent viral, permettant d'augmenter la production et/ou la stabilité du vecteur viral ou du rétrovirus, contenant le gène à transfecter.

18. Procédé, selon l'une des revendications 11 à 17, comprenant à l'issue de l'étape du transfert de tout ou partie du gène hétérologue, la culture des cellules souches hématopoïétiques comportant dans leur génome tout ou partie d'un gène hétérologue dans un milieu approprié, notamment un milieu liquide ou un milieu semi-solide, pour obtenir des cellules hématopoïétiques différenciées, notamment les cellules différenciées du sang.

19. Procédé selon l'une des revendications 11 à 17, comprenant à l'issue de l'étape de transfert de tout ou partie du gène hétérologue, une étape de remise à l'état de repos des cellules souches hématopoïétiques contenant tout ou partie du gène hétérologue, cette étape pouvant être effectuée à l'aide d'au moins un inhibiteur du cycle cellulaire, tel que le TGF-β.

20. Procédé de détermination du degré de maturité de cellules souches hématopoïétiques, **caractérisé en ce qu'**il comprend les étapes suivantes :
- on effectue la culture d'un premier ensemble de cellules souches hématopoïétiques, dans un milieu approprié à leur culture, mais ne contenant pas de moyens de blocage d'au moins un inhibiteur du cycle cellulaire, pendant environ 14 à environ 28 jours, de préférence 18 jours,
- on effectue la culture d'un deuxième ensemble de cellules souches hématopoïétiques, de même nature et de même degré de maturité que celles mentionnées ci-dessus, dans un milieu approprié contenant des moyens de blocage d'au moins un inhibiteur du cycle cellulaire, ces moyens de blocage étant présents, dans le milieu de culture à une concentration efficace, pendant une période n'excédant pas 72 h, de préférence d'environ 1 à 15 h et de préférence encore, de 1 à 10 h, cette culture ayant lieu pendant la même durée que celle du premier ensemble de cellules souches,
- on compare, 18 jours après la mise en culture de chacun des deux ensembles de cellules souches, le nombre et la nature des colonies, la différence des sommes des colonies à haut potentiel prolifératif (HPP-CFC, HPP-MEG, HPP-GEMM) respectivement entre celle du deuxième ensemble et celle du premier ensemble correspondant aux colonies HPP-Q.

## Patentansprüche

1. Verwendung von Hemmstoffen von wenigstens einem Inhibitor des Zellzyklus über eine Zeitspanne, die 72 h nicht übersteigt und bevorzugt 36 h nicht übersteigt, und insbesondere geringer als etwa 20 h und bevorzugt etwa 1 bis 15 h und bevorzugt etwa 1 bis 10 h ist, in einer Kultur von Zellen einer hämatopoetischen Zelllinie, um die Zellen der hämatopoetischen Zelllinie aus ihrem Ruhezustand zu befreien.

2. Verwendung der Hemmstoffe von wenigstens einem Inhibitor des Zellzyklus nach Anspruch 1, in einer Vergleichsuntersuchung zwischen einerseits einer Kultur von Zellen einer hämatopoetischen Zelllinie in einem Medium, welches die oben genannten Hemmstoffe von wenigstens einem Inhibitor des Zellzyklus enthält, und andererseits einer gleichen Kultur von Zellen einer hämatopoetischen Zelllinie wie die oben erwähnte, in einem Medium, welches keine der oben genannten Hemmstoffe von wenigstens einem Inhibitor des Zellzyklus enthält, um den Reifegrad der oben genannten Zellen einer hämatopoetischen Zelllinie zu bestimmen.

3. Verwendung der Hemmstoffe von wenigstens einem Inhibitor des Zellzyklus nach Anspruch 1 in einer Kultur von Zellen einer hämatopoetischen Zelllinie, um die Zellen einer hämatopoetischen Zelllinie aus ihrem Ruhezustand zu befreien, gefolgt von einem Gentransferschritt in die oben genannten Zellen einer hämatopoetischen Zelllinie.

4. Verwendung nach einem der Ansprüche 1 bis 3, wobei die Zellen der Zelllinie hämatopoetische Zellen sind, und wobei die Hemmstoffe mit wenigstens einem Inhibitor des Zellzyklus aus dem anti-TGF-β bestehen.

5. Zellen einer hämatopoetischen Zelllinie, welche ein vollständiges oder einen Teil eines heterologen Gens in ihrem Genom enthalten, welche durch das Verfahren erhalten werden können, das die folgenden Schritte umfasst:
- Vorstimulation der Zellen einer hämatopoetischen Zelllinie im Ruhezustand in einem Medium, welches enthält:
* direkte oder indirekte Hemmstoffe von wenigstens einem Inhibitor des Zellzyklus der besagten Zellen einer hämatopoetischen Zelllinie über eine ausreichende Zeitspanne, um die Zellen einer hämatopoetischen Zelllinie aus ihrem Ruhezustand zu befreien, und die 72 h nicht übersteigt und bevorzugt 36 h nicht übersteigt, und insbesondere geringer als etwa 20 h und bevorzugt etwa 1 h bis 15 h und bevorzugt etwa 1 h bis 10 h beträgt, und
* Substanzen, insbesondere Zytokine, um die Aktivierung in einen Zellzyklus und/oder das spätere Durchlaufen wenigstens eines Zellzyklus zu ermöglichen,
- den Transferschritt durch Vorlegen der im vorherigen Schritt erhaltenen Zellen einer hämatopoetischen Zelllinie mit einem vollständigen oder einen Teil eines heterologen Gens in einem Medium, welches den Transfer des vollständigen oder eines Teils des heterologen Gens in das Genom der Zellen einer hämatopoetischen Zelllinie ermöglicht, um Zellen einer hämatopoetischen Zelllinie zu erhalten, welche vollständig oder einen Teil eines heterologen Gens enthalten,
- die eventuelle Rückgewinnung der durch den vorhergehenden Schritt erhaltenen Zellen einer hämatopoetischen Zelllinie.

6. Zellen einer hämatopoetischen Zelllinie nach Anspruch 5, welche in ihrem Genom ein vollständiges oder einen Teil eines heterologen Gens enthalten, und ein Multiplikationspotential von gleich oder mehr als 10⁶, und ein Differenzierungspotential haben, welches sich über die gesamten hämatopoetischen Zelllinien erstreckt.

7. Zellen einer hämatopoetischen Zelllinie nach Anspruch 5 oder 6, welche in ihrem Innern oder auf ihrer Oberfläche Hemmstoffe mit einem vollständigen Inhibitor ihres Zellzyklus aufweisen.

8. Zellen nach einem der Ansprüche 5 bis 7, welche sich im Ruhezustand befinden.

9. Zellen einer hämatopoetischen Zelllinie nach einem der Ansprüche 5 bis 8, in denen die Hemmstoffe ausgewählt sind aus: Den anti-Inhibitoren des Zellzyklus, zum Beispiel Hemmstoffe der Tumorsuppressorgene, Antagonisten des TGF-β, wie TGF-β-Antisense, neutralisierende Antikörper gegen TGF-β, lösliche Rezeptoren des TGF-β oder natürliche oder synthetische Inhibitoren des TGF-β oder Inhibitoren des Syntheseweges von TGF-β.

10. Zellen einer hämatopoetischen Zelllinie nach einem der Ansprüche 5 bis 9, wobei das vollständige oder ein Teil des heterologen Gens aus den folgenden Genen ausgewählt werden kann: Marker: Neo (Gen der Neomycinresistenz), CD2 (Leukozytenantigen), nls-Lac-Z, oder Gene, die eine Hämatopathie oder eine andere Krankheiten heilen können, wie etwa ADA (Gen der Adenosindesaminase), ALDP (Gen, das Adrenoleukodystrophie heilen kann), TK (Suizidgen unter der Kontrolle viraler Promotoren, das es ermöglicht, Zellen in der Anwesenheit von Aciclovir zu töten).

11. Verfahren zum Transfer eines vollständigen oder eines Teils eines heterologen Gens in das Genom von Zellen einer hämatopoetischen Zelllinie, welche aus dem Ruhezustand aktiviert wurden, **dadurch gekennzeichnet, dass** es die folgenden Schritte umfasst:
- Vorstimulation der Zellen einer hämatopoetischen Zelllinie im Ruhezustand in einem Medium, welches enthält:
* direkte oder indirekte Hemmstoffe von wenigstens einem Inhibitor des Zellzyklus der besagten Zellen einer hämatopoetischen Zelllinie über eine ausreichende Zeitspanne, um die Zellen einer hämatopoetischen Zelllinie, aus ihrem Ruhezustand zu befreien, und die 72 h nicht übersteigt und bevorzugt 36 h nicht übersteigt, und insbesondere geringer als etwa 20 h und bevorzugt etwa 1 h bis 15 h und bevorzugt etwa 1 h bis 10 h beträgt, und
* Substanzen, insbesondere Zytokine, um die Aktivierung in einen Zellzyklus und/oder das spätere Durchlaufen wenigstens eines Zellzyklus zu ermöglichen,
- den Transferschritt durch Vorlegen der im vorherigen Schritt erhaltenen Zellen einer hämatopoetischen Zelllinie mit einem vollständigen oder eines Teils eines heterologen Gens in einem Medium, welches den Transfer des vollständigen oder eines Teils des heterologen Gens in das Genom der Zellen einer hämatopoetischen Zelllinie ermöglicht, um Zellen einer hämatopoetischen Zelllinie zu erhalten, welche vollständig oder einen Teil eines heterologen Gens enthalten,
- die eventuelle Rückgewinnung der durch den vorhergehenden Schritt erhaltenen Zellen einer hämatopoetischen Zelllinie.

12. Verfahren nach Anspruch 11, wobei der Transferschritt eines vollständigen oder eines Teils eines heterologen Gens durch eine Kokultivierung von gemäß dem ersten Schritt des Anspruchs 11 vorstimulierten Zellen einer hämatopoetischen Zelllinie und von Zellen, welche einen Vektor produzieren, wie einen ein vollständiges oder einen Teil eines zu transferierenden Gens umfassender viraler Vektor oder Retrovirus, erfolgt.

13. Verfahren nach Anspruch 11, wobei der Transferschritt des vollständigen oder eines Teils des heterologen Gens durch Vorlegen von gemäß dem ersten Schritt des Anspruchs 11 vorstimulierten Zellen einer hämatopoetischen Zelllinie und durch Überschichten mit Zellen, welche einen Vektor produzieren, wie einen ein vollständiges oder einen Teil eines zu transferierenden Gens umfassender viraler Vektor oder Retrovirus, erfolgt.

14. Verfahren nach einem der Ansprüche 11 bis 13, wobei das vollständige oder ein Teil eines zu transferierenden Gens aus einem Vektor wie einem viralen Vektor oder einem Retrovirus stammt, welcher durch eine produktive Kultur produziert wird, zu der ein virales Mittel, wie ein anti-TGF-β, ein anti-Interferon oder alle vektorstabilisierenden Mittel, hinzugegeben wird.

15. Verfahren nach einem der Ansprüche 11 bis 14, wobei das vorstimulierende Medium umfasst: IMDM (Iscove Dulbecco Modified Medium), Stem GEM* (welches einem vom C.N.R.S. vertriebenen Medium entspricht), Zytokine, Antagonisten des TGF-β, wie TGF-β-Antisense-Oligonukleotide, neutralisierende Antikörper gegen das TGF-β, lösliche Rezeptoren des TGF-β oder natürliche oder synthetische Inhibitoren des TGF-β, Mischungen von Zytokinen, welche geeignet sind, über einen kurzen Zeitraum (weniger als 72 h, bevorzugt weniger als 10 h) zu hemmen, die Inhibitoren des Zellzyklus der ruhenden Zellen der Zelllinie.

16. Verfahren nach einem der Ansprüche 11 bis 15, wobei die Substanzen, welche die Aktivierung in einen Zellzyklus und/oder das spätere Durchlaufen wenigstens eines Zellzyklus erlauben, ausgewählt sind aus einer Mischung von wenigstens 4 Substanzen, ausgewählt aus den Folgenden: IL1, IL2, IL3, IL4, IL5, IL6, IL7, IL11, GM-CSF (Granulozyten-Monozyten Kolonien stimulierender Faktor), G-CSF, Steel-Faktor (SF), ein Ligand von FLT3 (FL), Erythropoetin, TNF, LIF, Thrombopoetin und Insulin oder Synthokine der Synthese.

17. Verfahren nach einem der Ansprüche 11 bis 16, wobei das Medium, welches den Transfer des vollständigen oder eines Teils des heterologen Gens erlaubt, besteht aus oder umfasst: DMEM und eventuell IMDM, Zytokine und eventuell einen Antagonisten des TGF-β, anti-Interferone, oder alle anderen viralen Mittel, welche die Produktion und/oder die Stabilität des das zu transfizierende Gen enthaltenden viralen Vektors oder Retrovirus erhöhen.

18. Verfahren nach einem der Ansprüche 11 bis 17, welches am Ende des Transferschritts des vollständigen oder eines Teils des heterologen Gens, die Kultur der Zellen einer hämatopoetischen Zelllinie, die in ihrem Genom ein vollständiges oder einen Teil eines heterologen Gens enthalten, in einem geeigneten Medium umfasst, insbesondere einem flüssigen Medium oder einem halbfesten Medium, um differenzierte hämatopoetische Zellen zu erhalten, insbesondere differenzierte Blutzellen.

19. Verfahren nach einem der Ansprüche 11 bis 17, welches am Ende des Transferschritts des vollständigen oder eines Teils des Gens einen Schritt des Versetzen der Zellen einer hämatopoetischen Zelllinie, welche ein vollständiges oder einen Teil eines heterologen Gens enthalten, in den Ruhezustand umfasst, wobei dieser Schritt durch die Hilfe wenigstens eines Inhibitors des Zellzyklus, wie das TGF-β, erfolgen kann.

20. Verfahren zur Bestimmung des Reifegrades der Zellen einer hämatopoetischen Zelllinie, **dadurch gekennzeichnet, dass** es die folgenden Schritte umfasst:
- Kultivierung eines ersten Ansatzes von Zellen einer hämatopoetischen Zelllinie in einem zu ihrer Kultivierung geeigneten Medium, welches aber keine Hemmstoffe von wenigstens einem Inhibitor des Zellzyklus enthält, für etwa 14 bis 28 Tage, bevorzugt 18 Tage,
- Kultivierung eines zweiten Ansatzes von Zellen einer hämatopoetischen Zelllinie von gleicher Herkunft und mit gleichem Reifegrad wie die vorher erwähnten Zellen, in einem geeigneten Medium, welches einen Hemmstoffe von wenigstens einem Inhibitor des Zellzyklus enthält, wobei diese Hemmstoffe in dem Kulturmedium in einer wirkungsvollen Konzentration vorliegen, über eine Zeitspanne, die 72 h nicht übersteigt, bevorzugt etwa 1 bis 15 h und bevorzugter etwa 1 bis 10 h, wobei diese Kultur die gleiche Dauer hat wie die der Zellen des ersten Ansatzes der Zelllinie,
- 18 Tage nach Beginn der Kultivierung jedes der beiden Zellansätze Vergleich zwischen den Zellen des zweiten Zellansatzes bzw. des ersten Zellansatzes hinsichtlich der Anzahl und des Zustands der Kolonien und des Unterschieds in der Menge der Kolonien mit hohem Proliferationspotential (HPP-CFC, HPP-MEG, HPP-GEMM), welche den HPP-Q-Kolonien entsprechen.

## Claims

1. Use of means of blocking at least one inhibitor of the cell cycle of the said cells, in particular of the said hematopoietic stem cells, for a period not exceeding 72 h, and preferably not exceeding 36 h, and in particular less than approximately 20 h, and preferably approximately 1 to 15 h, and preferably approximately 1 to 10 h, in a culture of hematopoietic stem cells, in order to release the hematopoietic stem cells from their quiescent state.

2. Use according to claim 1 of means of blocking at least one inhibitor of the cell cycle in test comparing, on the one hand, a culture of hematopoietic stem cells in a medium which contains the aforementioned means of blocking at least one inhibitor of the cell cycle and, on the other hand, the same culture of hematopoietic stem cells as that mentioned above in a medium which does not contain the aforementioned means of blocking at least one inhibitor of the cell cycle, in order to determine the degree of maturity of the aforementioned hematopoietic stem cells.

3. Use according to claim 1 of means of blocking at least one inhibitor of the cell cycle in a culture of hematopoietic stem cells, in order to release the hematopoietic stem cells from their quiescent state, followed by a step in which genes are transferred into the aforementioned hematopoietic stem cells.

4. Use according to one of claims 1 to 3 in which the stem cells are hematopoietic stem cells and the means of blocking at least one inhibitor of the cell cycle consist in anti-TGF-β.

5. Hematopoietic stem cells, containing all or part of a heterologous gene in their genome, likely to be obtained according to the method comprising the following steps:
- the prestimulation of quiescent hematopoietic stem cells, in a medium containing:
* direct or indirect means of blocking at least one inhibitor of the cell cycle of the said hematopoietic stem cells, for a period which is sufficient to release the hematopoietic stem cells, from their quiescent state, and does not exceed 72 h, preferably does not exceed 36 h, and in particular less than approximately 20 h, and preferably approximately 1 h to 15 h, and preferably approximately 1 h to 10 h, and
* substances, in particular cytokins, for permitting the activation of a cell cycle and/or subsequently the passage of at least one cell cycle,
- the step comprising transfer by contacting the hematopoietic stem cells, obtained in the previous step, with all or part of a heterologous gene, in a medium permitting the transfer of all or part of the heterologous gene into the genome of the hematopoietic stem cells, in order to obtain hematopoietic stem cells, including all or part of a heterologous gene,
- the possible recovery of the hematopoietic stem cells, obtained in the previous step.

6. Hematopoietic stem cells according to claim 5, containing in their genome all or part of a heterologous gene and having a multiplication potential equal to or greater than 10⁶ and a differentiation potential likely to cover all the hematopoietic lineages.

7. Hematopoietic stem cells according to one of claims 5 or 6, including, inside themselves or on their surface, means of blocking any inhibitor of their cell cycle.

8. Cells according to any one of claims 5 to 7, in a quiescent state.

9. Hematopoietic stem cells according to one of claims 5 to 8, in which the blocking means comprise of those chosen from among: anti-inhibitors of the cell cycle, for example agents blocking tumor-suppressing genes, antagonists of TGF-β such as antisenses of TGF-β, antibodies neutralizing TGF-β, soluble receptors of TGF-β or natural or synthetic inhibitors of TGF-β, or inhibitors of the TGF-β synthesis path.

10. Hematopoietic stem cells according to one of claims 5 to 9, in which all or part of the heterologous gene can be chosen from among the following genes: markers: Neo (neomycin resistance gene), CD2 (leucocytic antigen), nls-lacZ, or genes correcting a hematopathy or any other pathology such as ADA (adenosine deaminase gene), ALDP (gene correcting adrenoleucodystrophy), TK (suicide gene allowing cells to be killed in the presence of acyclovir under the control of viral promoters).

11. Method of transferring all or part of a heterologous gene into the genome of hematopoietic stem cells activated from a quiescent state, **characterized in that** it comprises the following steps:
- the prestimulation of quiescent hematopoietic stem cells, in a medium containing:
* direct or indirect means of blocking at least one inhibitor of the cell cycle of the said hematopoietic stem cells for a period which is sufficient to release the hematopoietic stem cells, from their quiescent state, and does not exceed 72 h, and preferably does not exceed 36 h, and in particular less than approximately 20 h and preferably approximately 1 h to 15 h, and preferably approximately 1 h to 10 h, and
* substances, in particular cytokines, for permitting the activation of a cell cycle and/or subsequently the passage of at least one cell cycle,
- the step comprising transfer by contacting the hematopoietic stem cells obtained in the previous step, and all or part of a heterologous gene, in a medium permitting the transfer of all or part of the heterologous gene into the genome of the hematopoietic stem cells, in order to obtain hematopoietic stem cells, including all or part of a heterologous gene,
- the possible recovery of the hematopoietic stem cells obtained in the previous step.

12. Method according to claim 11, in which the step comprising the transfer of all or part of the heterologous gene takes place with a co-culture of hematopoietic stem cells, prestimulated according to the first step of claim 11, and cells producing a vector, such as a viral vector or a retrovirus, containing all or part of the gene to be transferred.

13. Method according to claim 11, in which the stage comprising the transfer of all or part of the heterologous gene takes place by bringing together hematopoietic stem cells, prestimulated according to the first step of claim 11, and the supernatant of cells containing a vector, such as a viral vector or a retrovirus containing all or part of the gene to be transferred.

14. Method according to one of claims 11 to 13, in which all or part of the gene to be transferred comes from a vector, such as a viral vector or a retrovirus produced by a producer culture to which is added a viral agent such as an anti-TGF-β, an anti-interferon, or any vector-stabilizing agent.

15. Method according to one of claims 11 to 14, in which the prestimulation medium comprises: IMDM (Iscove Dubelcco Modified Medium), Stem GEM* (corresponding to a medium marketed by the C.N.R.S.), cytokins, antagonists of TGF-β such as antisense oligonucleotides of TGF-β, antibodies neutralizing TGF-β, soluble receptors of TGF-β or natural or synthetic inhibitors of TGF-β, combinations of cytokins likely to block in a short time space (less than 72 h, preferably less than 10 h) the inhibitors of the cell cycle of the quiescent stem cells.

16. Method according to one of claims 11 to 15, in which the substances permitting the activation of a cell cycle and/or subsequently the passage of at least one cell cycle are chosen from among a combination of at least 4 substances chosen from among the following: IL1, IL2, IL3, IL4, EL5, IL6, IL7, IL11, GM-CSF (granulocyte-monocyte colony-stimulating factor), G-CSF, Steel factor (SF), ligand of FLT3 (FL), erythropoietin, TNF, LIF, thrombopoietin, and insulin or synthetic synthokins.

17. Method according to one of claims 11 to 16, in which the medium permitting the transfer of all or part of the heterologous gene is comprised of, or contains: DMEM, and possibly IMDM, cytokins, and possibly an antagonist of TGF-β, anti-interferons, or any other viral agent, permitting an increase in the production and/or the stability of the viral vector or of the retrovirus containing the gene to be transfected.

18. Method according to one of claims 11 to 17, comprising, at the end of the stage transferring all or part of the heterologous gene, the culture of the hematopoietic stem cells including in their genome all or part of a heterologous gene in a suitable medium, in particular a liquid medium or a semi-solid medium, in order to obtain differentiated hematopoietic cells, in particular differentiated blood cells.

19. Method according to one of claims 11 to 17, comprising, at the end of the stage transferring all or part of the heterologous gene, a step comprising the returning to a quiescent state of the hematopoietic stem cells containing all or part of the heterologous gene, this step being capable of being carried out with the help of at least one inhibitor of the cell cycle, such as TGF-β.

20. Method of determining the degree of maturity of hematopoietic stem cells, **characterized in that** it comprises the following steps:
- a first set of hematopoietic stem cells is cultured in a medium which is suitable for their culture, but does not contain means of blocking at least one inhibitor of the cell cycle, for approximately 14 to approximately 28 days, preferably 18 days,
- a second set of hematopoietic stem cells is cultured, of the same kind and having the same degree of maturity as those mentioned above, in a suitable medium which contains means of blocking at least one inhibitor of the cell cycle, these blocking means being present in the culture medium in an effective concentration for a period which does not exceed 72 h, preferably approximately 1 to 15 h and more preferably from 1 to 10 h, this culture taking place for the same period as that of the first set of stem cells,
- a comparison is carried out, 18 days after the culturing of each of the two sets of stem cells, of the number and type of colonies, of the difference in the totals of the colonies having a high proliferative potential (HPP-CFC, HPP-MEG, HPP-GEMM) respectively between that of the second set and that of the first set corresponding to the HPP-Q colonies.
